# EUROPEAN PATENT APPLICATION

(11) **EP 4 817 660 A2**
(43) Date of publication of application: **07.10.2026**
(21) Application number: 26188263.3
(22) Date of filing: 07.03.2022
(51) Int. Cl.: A61M 16/00

(54) **ALARM FOR BREATHING ASSISTANCE SYSTEM**

(30) Priority: 08.03.2021 US 202163158088 P
(62) Document number(s) of the earlier application(s) in accordance with Art. 76 EPC: 22766469.5 / 4 304 686
(71) Applicant: Fisher & Paykel Healthcare Limited, East Tamaki, Auckland 2013 (NZ)
(72) Inventor: GULLEY, Anton Kim, 2013 Auckland (NZ); ELLINGHAM, Lucy Elizabeth, 2013 Auckland (NZ)
(74) Representative: Gill Jennings & Every LLP

(57) **Abstract**

Disclosed is a breathing apparatus, the breathing apparatus comprising a flow generator, a flow path, a and a controller. The flow path may be configured to provide a flow of breathable gas to a patient. The controller may be configured to control the flow generator by controlling a flow generator output to the flow generator the controller may be configured to determine a predicted flow rate for the flow path, and/or a predicted maximum flow rate for the flow path and/or a predicted minimum flow rate for the flow path.

## Description

### Field of the invention

The present invention relates to methods and systems for controlling and/or operating a breathing assistance apparatus.

### Background

Breathing assistance apparatuses are used to deliver a flow of gas to patients in various environments (such as hospital, medical facility, residential care, or home environments). A breathing assistance apparatus (e.g. a flow therapy apparatus) may include an oxygen inlet that enables the respiratory apparatus to deliver supplemental oxygen with the flow of gas. A breathing assistance apparatus may also (or alternatively) include a humidification apparatus that enables the respiratory apparatus to deliver heated and humidified gases. A breathing assistance apparatus may allow adjustment of, and control over, characteristics of the gases flow. These characteristics may include for example flow rate, temperature, gas concentration (such as supplemental oxygen concentration), humidity, and pressure, etc.

Patients suffering from various health conditions and diseases can benefit from respiratory therapy. In at least one form, the respiratory therapy may be oxygen therapy. For example, a patient suffering from chronic obstructive pulmonary disease (COPD), pneumonia, asthma, bronchopulmonary dysplasia, heart failure, cystic fibrosis, sleep apnea, lung disease, trauma to the respiratory system, acute respiratory distress, and/or other conditions or diseases can benefit from respiratory therapy. Similarly, patients receiving pre- and post- operative oxygen delivery can also benefit from respiratory therapy.

### Summary

In an aspect of the disclosure there is provided a breathing apparatus, the breathing apparatus comprising:
a flow generator
a flow path, the flow path configured to provide a flow of breathable gas to a patient,
a controller, the controller configured to control the flow generator by controlling a flow generator output to the flow generator, wherein the flow generator output is within an allowable flow generator output range, to provide the flow of breathable gas, and
wherein the controller is configured to:
   determine a variable indicative of conductance of the flow path,
   determine a predicted maximum flow rate and/or a predicted minimum flow rate for the flow path based on:
      the variable indicative of conductance of the flow path,
      the allowable flow generator output range.

In some embodiments, the flow generator output is or comprises:
i. a motor speed,
ii. a motor current,
iii. a motor duty,
iv. a pressure of the gases in the flow path, or
v. any combination of i-iv.

In some embodiments, the variable indicative of conductance of the flow path is, or is based on:
i. a conductance of the flow path, or a portion of the flow path;
ii. a resistance to flow of the flow path, or a portion of the flow path; or
iii. a pressure drop along the flow path, or a portion of the flow path.
iv. any combination of i-iii.

In some embodiments, the flow generator comprises an impeller.

In some embodiments, the breathing apparatus comprises a flow sensor, wherein the flow sensor is configured to measure a flow rate of the flow of breathable gas to a patient.

In some embodiments, the variable indicative of conductance of the flow path is based on a variable indicative of conductance of the flow path at a previous time step.

In some embodiments, the variable indicative of conductance of the flow path is based on a variable indicative of conductance of the flow path over a timing window, optionally the timing window is a preceding 20 seconds.

In some embodiments, the variable indicative of conductance of the flow path is based on the current flow generator output and a current flow rate.

In some embodiments, the variable indicative of conductance of the flow path is based on the current flow divided by the current flow generator.

In some embodiments, the variable indicative of conductance is proportional to: $\frac{flow rate}{\sqrt{pressure}}$

In some embodiments, the variable indicative of conductance is proportional to: $\frac{flow rate}{motor output}$

In some embodiments, the flow path comprises one or more of:
a humidification chamber,
one or more breathing conduits,
a patient interface,
an elbow, and
a sensor module.

In some embodiments, the flow path may be downstream of the flow generator (and optionally the output of the flow generator).

In some embodiments, the flow path may be downstream of the humidifier (and optionally the output of the humification chamber).

In some embodiments, the flow path may be downstream of an or the elbow.

In some embodiments, the flow path may be downstream of a or the sensor module.

In some embodiments, the flow path may comprise at least a humidification chamber, a patient interface and a conduit (and optionally a sensor module).

In some embodiments, the flow path may comprise at least a patient interface and a conduit (and optionally a sensor module).

In some embodiments, the predicted maximum flow rate and/or the predicted minimum flow is determined periodically (optionally at each time step).

In some embodiments, the controller configured to control the motor to provide, or attempt to provide, the flow of breathable gas to the patient at a flow rate set point.

In some embodiments, the allowable flow generator output range is based on the flow rate set point.

In some embodiments, the allowable flow generator output range is an allowable motor speed range, wherein the allowable motor speed range comprises an upper motor speed limit and/or a lower motor speed limit.

In some embodiments, the predicted maximum flow rate is based on the current motor speed and/or an upper motor speed limit.

In some embodiments, the predicted maximum flow rate is based on a difference between the current motor speed and an upper motor speed limit.

In some embodiments, the predicted minimum flow rate is based on the current motor speed and/or a lower motor speed limit.

In some embodiments, the predicted minimum flow rate is based on a difference between the current motor speed and a lower motor speed limit.

In some embodiments, the controller is configured to compare the predicted maximum flow rate and/or the predicted minimum flow rate, and a flow rate set point, and based on the comparison generate at least one alarm condition.

In some embodiments, the at least one alarm condition comprises one or more of:
a cannot reach flow rate set point alarm, and
a blockage alarm.

In some embodiments, the cannot reach flow rate set point alarm comprises:
a cannot reach flow rate alarm associated with the flow rate set point being above the maximum predicted flow rate (i.e. a flow rate set point too high alarm), and/or
a cannot reach flow rate alarm associated with the flow rate set point being below the minimum predicted flow rate (i.e. a flow rate set point too low alarm).

In some embodiments, the controller is configured to determine a condition where the flow rate set point cannot be reached, based on the predicted maximum flow rate and the flow rate set point, and in response, the controller is configured to generate a cannot reach flow rate set point alarm.

In some embodiments, the controller is configured to generate at least one alarm condition when the flow rate set point is greater than the predicted maximum flow rate (optionally the alarm condition is a cannot reach flow rate set point alarm i.e. a flow rate set point too high alarm).

In some embodiments, the controller is configured to generate at least one alarm condition (optionally the alarm condition is a cannot reach flow rate set point alarm i.e. a flow rate set point too high alarm) when the flow rate set point is greater than the predicted maximum flow rate by more than an alarm threshold.

In some embodiments, the alarm threshold is a proportion of the flow rate set point (optionally the alarm condition is a cannot reach flow rate set point alarm i.e. a flow rate set point too high alarm).

In some embodiments, the proportion of the flow rate set point is about 5% to about 20% or about 10% of the flow rate set point.

In some embodiments, the alarm threshold is based on a type of therapy being provided by the breathing apparatus.

In some embodiments, when the flow rate set point is greater than the predicted maximum flow rate by more than a blockage threshold the controller is configured to generate a blockage alarm.

In some embodiments, the blockage threshold is about 20% to about 70%, or about 50% of the flow rate set point.

In some embodiments, the controller is configured to determine a condition where flow rate set point cannot be reached, based on the predicted minimum flow rate and the flow rate set point, and in response the controller is configured to generate a cannot reach flow rate set point alarm.

In some embodiments, the controller is configured to generate at least one alarm condition when the flow rate set point is less than the predicted minimum flow rate (optionally the alarm condition is a cannot reach flow rate set point alarm i.e. a flow rate set point too low alarm).

In some embodiments, the controller is configured to generate at least one alarm condition (optionally the alarm condition is a cannot reach flow rate set point alarm i.e. a flow rate set point too low alarm) when the flow rate set point is less than the predicted minimum flow rate by more than an alarm threshold.

In some embodiments, the alarm threshold is a proportion of the flow rate set point (optionally the alarm condition is a cannot reach flow rate set point alarm).

In some embodiments, the proportion of the flow rate set point is about 5% to about 20% or about 10% of the flow rate set point.

In some embodiments, the alarm threshold is based on a type of therapy being provided by the breathing apparatus.

In some embodiments, in response to the generation of the at least one alarm condition, the controller controls the motor speed to a predetermined safe motor speed.

In some embodiments, the controller controls the motor speed to the predetermined safe motor speed until the predicted maximum flow rate is greater than a safe threshold flow rate, and/or the predicted minimum flow rate is less than a safe threshold flow rate.

In some embodiments, the controller controls the motor speed to the predetermined safe motor speed until the flow rate set point is less than the predicted maximum flow rate, and/or the flow rate set point is greater than the predicted minimum flow rate.

In some embodiments, the predetermined safe motor speed is 8000RPM.

In some embodiments, the alarm condition comprises one or more of:
an audio alarm, and
a visual alarm.

In some embodiments, the breathing apparatus is a nasal high flow apparatus.

In some embodiments, the breathing apparatus provides the flow of gases to a patient via a non-sealing interface.

In another aspect of the disclosure there is provided a breathing apparatus, the breathing apparatus comprising:
a flow generator,
a flow path, the flow path configured to provide a flow of breathable gas to a patient,
a controller, the controller configured to control the flow generator by controlling a flow generator output to the flow generator, to provide the flow of breathable gas to a patient, and
   wherein the controller is configured to:
   determine a variable indicative of conductance of the flow path, and
   determine a predicted flow rate for the flow path based on:
      the variable indicative of conductance of the flow path and
      the current flow generator output.

In some embodiments, at a first time step, the controller is configured to determine a variable indicative of conductance of the flow path.

In some embodiments, at a second time step, the controller is configured to determine a predicted flow rate based on the variable indicative of conductance of the flow path.

In some embodiments, the flow generator output is or comprises:
i. a motor speed,
ii. a motor current,
iii. a motor duty,
iv. a pressure of the gases in the flow path, or
v. any combination of i-iv.

In some embodiments, the variable indicative of conductance of the flow path is, or is based on:
i. a conductance of the flow path, or a portion of the flow path;
ii. a resistance to flow of the flow path, or a portion of the flow path; or
iii. a pressure drop along the flow path, or a portion of the flow path.
iv. any combination of i-iii.

In some embodiments, the controller is configured to compare a current flow rate and the predicted flow rate, and based on the comparison generate at least one alarm condition.

In some embodiments, the controller is configured to compare a current flow rate and at least one flow rate threshold, and based on the comparison generate at least one alarm condition.

In some embodiments, the controller is configured to generate a leak alarm condition if the current flow rate is greater than at least one leak flow rate threshold (optionally when the predicted flow rate is less than the current flow rate).

In some embodiments, the leak alarm condition is generated when the current flow rate is greater than all of the at least one leak flow rate threshold.

In some embodiments, the leak alarm condition is generated after a plurality of instances of the current flow rate being greater than the at least one leak flow rate threshold.

In some embodiments, the at least one leak flow rate threshold comprises one or more of:
a percentage threshold based on a percentage of the predicted flow rate
a limit threshold based on the flow generator output and optionally a constant,
a variability threshold based on the variability of the predicted flow rate.

In some embodiments, if the current flow rate is greater than any combination of the percentage threshold, the limit threshold, and/or the variability threshold, the controller is configured to generate the leak alarm condition.

In some embodiments, the percentage of the predicted flow rate is about 105% to about 180%, or about 110% to about 120%, or about 150% to about 180%.

In some embodiments, the limit threshold is based on the flow generator output and a constant, wherein the constant is a predetermined conductance.

In some embodiments, the variability threshold is based on the standard deviation of the variable indicative of the conductance of the flow path.

In some embodiments, the variability threshold is based on the predicted flow rate.

In some embodiments, the variability threshold is based on an error in the mean of the variable indicative of the conductance of the flow path.

In some embodiments, the controller is configured to generate at least one blockage alarm condition if the current flow rate is less than the at least one blockage flow rate threshold.

In some embodiments, the blockage alarm condition is generated when the current flow rate is less than all of the at least one blockage flow rate threshold.

In some embodiments, the blockage alarm condition is generated after a plurality of instances of the current flow rate being less than the at least one blockage flow rate threshold.

In some embodiments, the at least one blockage flow rate threshold comprises one or more of:
a percentage threshold based on a percentage of the predicted flow rate
a limit threshold based on the flow generator output and optionally a constant,
a variability threshold based on the variability of the predicted flow rate.

In some embodiments, if the current flow rate is less than any combination of the percentage threshold, the limit threshold and/or the variability threshold, the controller is configured to generate the blockage alarm condition.

In some embodiments, the percentage of the predicted flow rate is about 40% to about 95%, or about 50% to about 90%, or about 60% to about 80% of the predicted flow rate
In some embodiments, the limit threshold is based on the flow generator output and a constant, wherein the constant is a predetermined conductance.

In some embodiments, the variability threshold is based on the standard deviation of the variable indicative of the conductance of the flow path.

In some embodiments, the variability threshold is based on the predicted flow rate.

In some embodiments, the variability threshold is based on an error in the mean of the variable indicative of the conductance of the flow path.

In some embodiments, in response to the generation of the at least one blockage alarm condition and/or the at least one leak alarm condition, the controller controls the motor speed to a predetermined safe motor speed.

In some embodiments, the controller controls the motor speed to the predetermined safe motor speed until at least one leak alarm condition or the at least one blockage alarm condition is cleared.

In some embodiments, the leak alarm condition and/or the blockage alarm condition comprises one or more of:
an audio alarm, and
a visual alarm.

In some embodiments, the variable indicative of conductance of the flow path may be represented by a probability density function.

In some embodiments, the variable indicative of conductance of the flow path is based on one or more weighting factors.

In some embodiments, the variable indicative of conductance of the flow path is based on conductance data, wherein the conductance data comprises at least one historic determination of the conductance of the flow path.

In some embodiments, the variable indicative of conductance of the flow path is based on conductance data of the flow path over a timing window, optionally the timing window is a preceding 20 seconds.

In some embodiments, at each timestep, or periodically, the determination of a conductance of the flow path is stored as the at least one historic determination of the conductance of the flow path.

In some embodiments, the conductance data comprises timestamp information associated with a respective historic determination of the conductance of the flow path.

In some embodiments, the timestamp information comprises a time that has elapsed since the historic determination of the conductance of the flow path was determined.

In some embodiments, the conductance data is weighted in the determination of the variable indicative of conductance of the flow path based on the associated timestamp information.

In some embodiments, more recent conductance data is given a larger weighting than less recent conductance data.

In some embodiments, the conductance data comprises a weighting factor associated with each respective historic determination of conductance of the flow path

In some embodiments, the controller is configured to determine a conductance of the flow path and determine a predicted flow rate for the flow path continuously, or periodically.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7).

Embodiments described herein can also be said broadly to relate to the parts, elements and features referred to or indicated in the specification of the application, individually or collectively, and any or all combinations of any two or more of said parts, elements or features, and where specific integers are mentioned herein which have known equivalents in the art to which this invention relates, such known equivalents are deemed to be incorporated herein as if individually set forth.

In this specification, where reference has been made to external sources of information, including patent specifications and other documents, this is generally for the purpose of providing a context for discussing the features of the present invention. Unless stated otherwise, reference to such sources of information is not to be construed, in any jurisdiction, as an admission that such sources of information are prior art or form part of the common general knowledge in the art.

### Brief Description of the Drawings

Figure 1 shows in diagrammatic form a breathing assistance apparatus.
Figure 2 illustrates a sensing circuit board that may be used in a breathing assistance apparatus.
Figure 3 is a first underside perspective view of the main housing of breathing assistance apparatus showing a recess inside the housing for the motor and/or sensor module sub-assembly.
Figure 3A is a second underside perspective view of the main housing of the breathing assistance apparatus showing the recess for the motor and/or sensor module sub-assembly.
Figure 4 is a perspective view of a breathing assistance apparatus.
Figure 5 is a perspective view of the motor and/or sensor subassembly, underside of the main housing, and fixed elbow of the breathing assistance apparatus.
Figure 6 is an exploded perspective view of components of the motor and/or sensor sub-assembly schematically showing by way of an arrow the gas flow path through the sub-assembly.
Figure 7 is an underside view of a cover and sensing PCB of the motor and/or sensor sub-assembly showing the position of sensors.
Figure 8 is a schematic gas flow path diagram for the filter module and the valve module, with the solid line arrows representing the flow
Figure 9 shows an example of the process for determining whether the flow rate set point is able to be reached.
Figure 10 illustrates an example of the relationship between the motor speed limits and the predicted flow rate.
Figure 11A, 11B1 and 11B2 show various comparisons between predicted minimum flow rates and flow rate set points.
Figure 11C shows an example of the process for determining whether a flow rate set point is able to be reached.
Figure 12A, 12B, 12C and 12D show various comparisons between predicted maximum flow rates and flow rate set points.
Figure 12E shows an example of the process for determining whether a flow rate set point is able to be reached and whether a blockage is present.
Figure 13 shows an example of the determining of whether a whether a flow rate set point is able to be reached and whether a blockage is present.
Figure 14 illustrates a method for determining a leak or blockage based on a predicted flow rate.
Figures 15A and 15B show illustrations of the leak flow thresholds plotted with the predicted flow rate and the current flow rate.
Figures 15C and 15D shows illustrations of the blockage flow thresholds plotted with the predicted flow rate and the current flow rate.
Figure 16 shows an example of a motor speed and associated predicted flow rate.
Figure 17 illustrates an example of a leak event and a number of leak flow rate thresholds.
Figure 18 illustrates an example of a blockage event and number of blockage flow rate thresholds

### Detailed Description

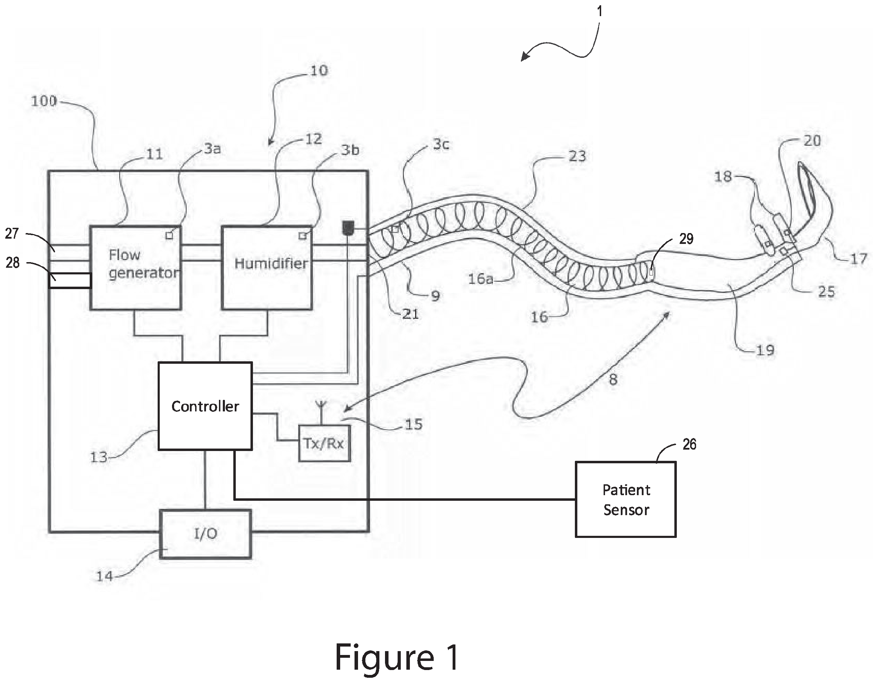

A breathing assistance apparatus 10 is shown in Figure 1. The breathing assistance apparatus 10 can comprise a main housing 100 that contains one or more of: a flow generator 11, which in some embodiments is in the form of a motor/impeller arrangement (for example, a blower), an optional humidifier 12, a controller 13, and a user interface 14 (comprising, for example, a display and input device(s) such as button(s), a touch screen, or the like).

In some embodiments, the humidifier 12 may comprise a humidification chamber. The humidification chamber may be configured to be removed from the humidifier (for example for replacement, cleaning and/or refilling). Alternatively, the humidification chamber may be integral to the humidifier.

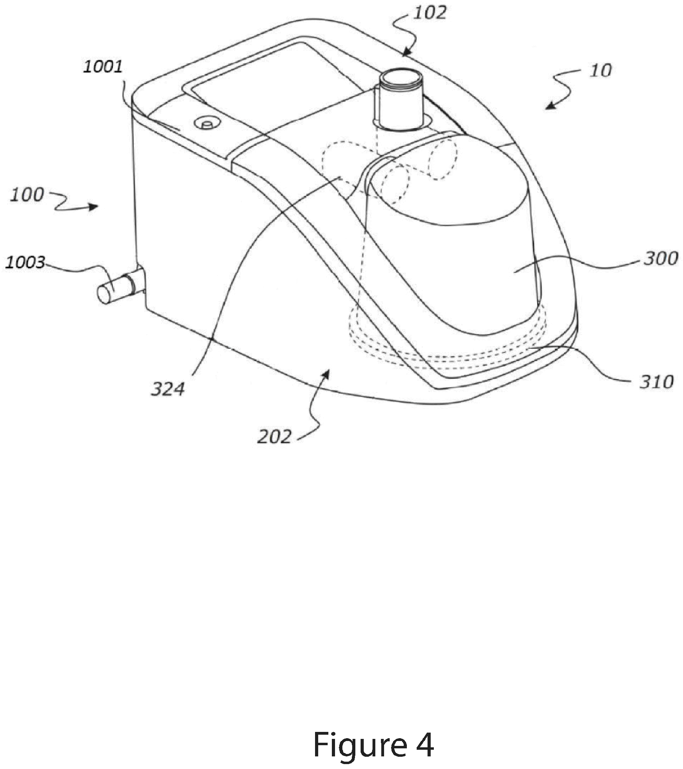

In some embodiments, the humidifier 12 may comprise a humidifier heating element 310 (see Figure 4). The humidifier heating element provides heat to the liquid chamber 300. The liquid in the liquid chamber may be water or another liquid, and/or may comprise a mixture one or more liquids (for example a mixture of water and a medicament.)

The humidifier 12 (if present) can humidify the gases flow and/or heat the gases flow to an appropriate level. The controller 13 can be configured to control the humidifier 12 (for example by controlling at least a humidifier heating element).

The controller 13 can be configured or programmed to control the operation of the breathing assistance apparatus 10. For example, the controller 13 can control components of the breathing assistance apparatus 10, including but not limited to: operating the flow generator 11 to create a flow of gas (gases flow) for delivery to a patient, operating the humidifier 12 (if present) to humidify and/or heat the generated gases flow, controlling a flow of oxygen into the flow generator blower, receiving user input from the user interface 14 for reconfiguration and/or user-defined operation of the breathing assistance apparatus 10, and outputting information (for example on the display) to the user.

The controller 13 may include one or more computer processors and associated non-transitory memory or storage mediums storing processor-executable instructions or code. The instructions, when executed by the one or more processors cause the respiratory therapy apparatus to affect the steps and processes described herein.

The term breathing assistance apparatus may be used interchangeably with respiratory assistance apparatus, or respiratory therapy apparatus or flow therapy apparatus.

The term breathing assistance system may be used interchangeably with respiratory assistance system, or respiratory therapy system, or flow therapy system.

The term current flow rate may refer to a measurement of a flow rate which has been presently made (for example at a current time step). It will be appreciated that the term current flow rate is not limited to the latest flow rate determination and could include recently made flow rate determinations (for example from a previous time step or the most recent flow rate determination), and/or a filtered flow rate determination made based on a series of past measurements (which may optionally include signal filtering and/or processing).

It will be appreciated the methods described herein can be executed by the controller (or another processor).

The methods described herein may be embodied as software or a software module as part of control software (for example computer-readable instructions) that are stored in the controller (or associated memory) and executed by the controller (and/or an associated processor).

The user can be one or more of a patient, healthcare professional (for example a clinician), or anyone else interested in using the apparatus.

As used herein, a "gases flow" can refer to any flow of gases that may be provided by the breathing assistance apparatus, such as a flow of ambient air, a flow comprising substantially 100% oxygen, a flow comprising some combination of ambient air and oxygen, and/or the like.

A patient breathing conduit 16 is coupled at one end to a gases flow outlet 21 in the housing 100 of the breathing assistance apparatus 10. The patient breathing conduit 16 is coupled at another end to a patient interface 17 such as a non-sealed nasal cannula with a manifold 19 and nasal prongs 18. Additionally, or alternatively, the patient breathing conduit 16 can be coupled to a face mask, a nasal mask, a nasal pillows mask, an endotracheal tube, a tracheostomy interface, and/or the like.

The gases flow that is generated by the breathing assistance apparatus 10 may be humidified and delivered to the patient via the patient breathing conduit 16 and the patient interface 17.

The patient breathing conduit 16 can have a heating element 16a to heat the gases flow passing through to the patient. The heating element 16a can be under the control of the controller 13. In at least one configuration, the heating element 16a is a heater wire. The patient breathing conduit 16 and/or patient interface 17 can be considered part of the breathing assistance therapy system. The breathing assistance system 1 may comprise the breathing assistance apparatus 10, patient breathing conduit 16, and patient interface 17.

The controller 13 can control the flow generator 11 to generate a gases flow at the desired flow rate. The controller 13 can also control a supplemental oxygen inlet to allow for delivery of supplemental oxygen.

The controller 13 can also control a humidifier heating element in the humidifier 12 and/or the heating element 16a in the patient conduit 16 to heat the gas to a desired temperature for a desired level of therapy and/or level of comfort for the patient.

The controller 13 can be provided with or can determine a suitable target temperature of the gases flow. The controller 13 may control the humidifier heating element in the humidifier 12 and/or the heating element 16b based on one or more suitable target temperature(s) of the gases flow.

In some embodiments the controller 13 may control the humidifier heating element in the humidifier 12 based on a set dew point at the patient interface and/or at the end of the inspiratory conduit.

In some embodiments the controller 13 may control the heating element 16b based on a set point temperature of the gases at the patient interface and/or at the end of the inspiratory conduit

As shown for example in Figure 4, the oxygen inlet port 28 includes a valve 1003 through which a pressurized gas may enter the respiratory therapy apparatus 10. The valve can control a flow of oxygen into the respiratory therapy apparatus 10. The valve can be any type of valve, including a proportional valve or a binary valve.

The source of oxygen can be an oxygen tank or a hospital oxygen supply. Medical grade oxygen is typically between 95% and 100% purity. Oxygen sources of lower purity can also be used. Examples of valve modules and filters are disclosed in WO2018074935, titled "Valve Module and Filter", filed on October 18, , which are hereby incorporated by reference in their entireties.

The breathing assistance apparatus 10 can measure and control the oxygen content of the gas being delivered to the patient, and therefore the oxygen content of the gas inspired by the patient.

In some embodiments, the breathing assistance apparatus 10 may provide high flow therapy, in which the high flow rate of gas delivered meets or exceeds the peak inspiratory demand of the patient.

Operation sensors 3a, 3b, 3c, such as flow, temperature, humidity, and/or pressure sensors can be placed in various locations in the breathing assistance apparatus 10. Additional sensors (for example, sensors 20, 25) may be placed in various locations on the patient conduit 16 and/or patient interface 17 (for example, there may be a temperature sensor 29 at or near the end of the inspiratory tube).

A flow path of gases through the apparatus 10 may be provided by one or more components of the system. For example, in Figure 1, the flow path of gases may be defined between the output of the flow generator 11 and the patient via the humidifier 12, conduit 16, patient interface 19 and any intermediate components. It will be appreciated that the flow path may be provided by different combinations of components of the system.

The respiratory therapy apparatus 10 may have a transmitter, receiver and/or transceiver 15 to enable the controller 13 to receive signals 8 from the sensors and/or to control the various components of the breathing assistance apparatus 10, including but not limited to the flow generator 11, humidifier 12, heating element 16a, humidifier heating element, or accessories or peripherals associated with the breathing assistance apparatus 10. Additionally, or alternatively, the transmitter, receiver and/or transceiver 15 may deliver data to a remote server or enable remote control of the respiratory therapy apparatus 10 or respiratory therapy system 1.

In some embodiments, the transmitter, receiver and/or transceiver 15 may be provided as, or as part of, a modem. The transmitter, receiver and/or transceiver 15 may use one or more communication protocols known in the art, for example Bluetooth, cellular (3G, 4G, or 5G etc).

In some embodiments, one or more leak or blockage events, and/or alarms (as described in more detail below) may be transmitted to one or more servers and/or devices (for example a computer, phone or tablet). Additional information (for example the time, duration, or severity) associated with the event or alarm may be additionally transmitted to the server and/or device.

In some embodiments, one or more leak or blockage events, and/or alarms (as described in more detail below) may be transmitted to a remote patient management system. The remote patient management system may be implemented a distributed computer system for example comprising one or more servers, and/or as a cloud based system. The one or more leak or blockage events, and/or alarms could be incorporated into a report. The report may be a patient report (i.e. specific to a patient), or related to a group of patients. The number of one or more leak or blockage events, and/or alarms may be included in the report along with other information (for example the associated time of the event and/or alarm).

As described above, the breathing assistance apparatus 10 can measure and control the oxygen content of the gas being delivered to the patient. Oxygen may be measured by placing one or more gas composition sensors (such as an ultrasonic transducer system) after the oxygen and ambient air have been mixed. The measurement can be taken within the respiratory therapy apparatus 10, the patient breathing conduit 16, the patient interface 17, or at any other suitable location.

The oxygen concentration measured in the device may be equivalent to the fraction of delivered oxygen (FdO2) and may be substantially the same as the oxygen concentration the patient is breathing, the fraction of inspired oxygen (FiO2), and as such the terms may be seen as equivalent.

Oxygen concentration may also be measured by using flow rate sensors on at least two of the ambient air inlet conduit, the oxygen inlet conduit, and the patient breathing conduit to determine the flow rate of at least two gases. By determining the flow rate of both inlet gases or one inlet gas and one total flow rate, along with the assumed or measured oxygen concentrations of the inlet gases (about 20.9% for ambient air, about 100% for oxygen), the oxygen concentration of the final gas composition can be calculated. Alternatively, flow rate sensors can be placed at all three of the ambient air inlet conduit, the oxygen inlet conduit, and the patient breathing conduit to allow for redundancy and testing that each sensor is working correctly by checking for consistency of readings. Other methods of measuring the oxygen concentration delivered by the breathing assistance apparatus 10 can also be used.

The breathing assistance apparatus 10 can include a patient sensor 26, such as a pulse oximeter or a patient monitoring system, to measure one or more physiological parameters of the patient, such as a patient's blood oxygen concentration (for example blood oxygen saturation (SpO2)), heart rate, respiratory rate, perfusion index, and provide a measure of signal quality. The sensor 26 can communicate with the controller 13 through a wired connection or by communication through a wireless transmitter on the sensor 26. The sensor 26 may be a disposable adhesive sensor designed to be connected to a patient's finger. The sensor 26 may be a non-disposable sensor (i.e. a re-useable sensor). Sensors that are designed for different age groups and to be connected to different locations on the patient are available and can be used with the breathing assistance system 1. The pulse oximeter can be attached to the patient, typically at their finger, although other places such as an earlobe are also an option. The pulse oximeter can be connected to a processor in the respiratory therapy apparatus 10 and can constantly provide signals indicative of the patient's blood oxygen saturation. The patient sensor 26 can be a hot-swappable device, which can be attached or interchanged during operation of the breathing assistance apparatus 10. For example, the patient sensor 26 may connect to the breathing assistance apparatus 10 using a USB interface or using wireless communication protocols (such as Bluetooth^{®}). When the patient sensor 26 is disconnected during operation, the breathing assistance apparatus 10 may continue to operate in its previous state of operation for a defined time period. After the defined time period, the breathing assistance apparatus 10 may trigger an alarm, transition from automatic mode to manual mode, and/or exit control mode (e.g., automatic mode or manual mode) entirely. The patient sensor 26 may be a bedside monitoring system or other patient monitoring system that communicates with the breathing assistance apparatus 10 through a physical or wireless interface.

The breathing assistance apparatus 10 may comprise or be in the form of a high flow therapy apparatus.

High flow therapy as discussed herein is intended to be given its typical ordinary meaning as understood by a person of skill in the art which generally refers to a breathing assistance apparatus delivering a targeted flow of humidified respiratory gases via an intentionally unsealed patient interface with flow rates generally intended to meet or exceed inspiratory flow of a patient. Typical patient interfaces include, but are not limited to, a nasal or tracheal patient interface. Typical flow rates for adults often range from, but are not limited to, about fifteen litres per minute to about sixty litres per minute or greater. Typical flow rates for paediatric patients (such as neonates, infants and children) often range from, but are not limited to, about one litre per minute per kilogram of patient weight to about three litres per minute per kilogram of patient weight or greater. High flow therapy can also optionally include gas mixture compositions including supplemental oxygen and/or administration of therapeutic medicaments. High flow therapy is often referred to as nasal high flow (NHF), humidified high flow nasal cannula (HHFNC), high flow nasal oxygen (HFNO), high flow therapy (HFT), or tracheal high flow (THF), among other common names.

For example, in some configurations, for an adult patient 'high flow therapy' may refer to the delivery of gases to a patient at a flow rate of greater than or equal to about 10 litres per minute (10 LPM), such as between about 10 LPM and about 100 LPM, or between about 15 LPM and about 95 LPM, or between about 20 LPM and about 90 LPM, or between about 25 LPM and about 85 LPM, or between about 30 LPM and about 80 LPM, or between about 35 LPM and about 75 LPM, or between about 40 LPM and about 70 LPM, or between about 45 LPM and about 65 LPM, or between about 50 LPM and about 60 LPM. In some configurations, for a neonatal, infant, or child patient, 'high flow therapy' may refer to the delivery of gases to a patient at a flow rate of greater than 1 LPM, such as between about 1 LPM and about 25 LPM, or between about 2 LPM and about 25 LPM, or between about 2 LPM and about 5 LPM, or between about 5 LPM and about 25 LPM, or between about 5 LPM and about 10 LPM, or between about 10 LPM and about 25 LPM, or between about 10 LPM and about 20 LPM, or between about 10 LPM and 15 LPM, or between about 20 LPM and 25 LPM. A high flow therapy apparatus with an adult patient, a neonatal, infant, or child patient, may, in some configurations, deliver gases to the patient at a flow rate of between about 1 LPM and about 100 LPM, or at a flow rate in any of the sub-ranges outlined above. Gases delivered may comprise a percentage of oxygen. In some configurations, the percentage of oxygen in the gases delivered may be between about 20% and about 100%, or between about 30% and about 100%, or between about 40% and about 100%, or between about 50% and about 100%, or between about 60% and about 100%, or between about 70% and about 100%, or between about 80% and about 100%, or between about 90% and about 100%, or about 100%, or 100%.

High flow therapy may be effective in meeting or exceeding the patient's inspiratory flow, increasing oxygenation of the patient, and/or reducing the work of breathing.

High flow therapy may be administered to the nares of a patient and/or orally, or via a tracheostomy interface.

High flow therapy may generate a flushing effect in the nasopharynx such that the anatomical dead space of the upper airways is flushed by the high incoming gases flow. This can create a reservoir of fresh gas available for each and every breath, while reducing re-breathing of nitrogen and carbon dioxide. Meeting inspiratory demand and flushing the airways is additionally important when trying to control the patient's FdO2. High flow therapy can be delivered with a non-sealing patient interface such as, for example, a nasal cannula. High flow therapy may slow down respiratory rate of the patient. High flow therapy may provide expiratory resistance to a patient.

The term "non-sealing patient interface" (i.e. unsealed patient interface) as used herein can refer to an interface providing a pneumatic link between an airway of a patient and a gases flow source (such as from flow generator 11) that does not completely occlude the airway of the patient. A non-sealed pneumatic link can comprise an occlusion of less than about 95% of the airway of the patient. The non-sealed pneumatic link can comprise an occlusion of less than about 90% of the airway of the patient. The non-sealed pneumatic link can comprise an occlusion of between about 40% and about 80% of the airway of the patient. The airway can include one or both nares of the patient and/or their mouth. For a nasal cannula the airway is through the nares.

The flow generator 11 can be or comprises a blower module. The blower module may comprise at least one blower 11 configured to generate said flow of gases.

The flow generator 11 can include an ambient air inlet port 27 through which ambient room air can be entrained into the blower. The breathing assistance apparatus 10 may also include an oxygen inlet port 28 leading to a valve through which a pressurized gas may enter the flow generator 11. The valve can control a flow of oxygen into the flow generator 11. The valve can be any type of valve, including a proportional valve or a binary valve.

The blower 11 can operate at a motor speed of greater than about 1,000 RPM and less than about 8,000 RPM, greater than about 2,000 RPM and less than about 10,000 RPM, or between any of the foregoing values. The blower 11 can mix the gases entering the blower 11 through the inlet ports (for example, the ambient air inlet port 27 and/or an oxygen inlet port 28). Using the blower 11 as the mixer can decrease the pressure drop relative to systems with separate mixers, such as static mixers comprising baffles.

The breathing assistance apparatus may further comprise a gas composition sensor. The gas composition sensor may be the sensor described below (for example the ultrasonic transducer configuration).

The breathing assistance apparatus 10 comprises a flow sensor. The flow sensor may be configured to measure a flow rate of the flow of breathable gas to a patient.

The controller 13 may comprise one or more processors. The processors may be configured with computer-readable instructions.

The controller 13 may comprise at least one memory element. The memory element may be configured to store said computer-readable instructions.

The memory element may be non-transitory.

The breathing assistance apparatus may comprise at least one display module, configured to display an alarm output.

The breathing assistance apparatus may comprise at least one audible module configured to emit an audible alarm.

In some embodiments the at least one audible module may comprise a speaker.

The display module may comprise at least one display (for example a liquid crystal display (LCD), or a light emitting diode (LED) display, although it will be appreciated any display technology may be used).

The display module may be a removeable display (for example a display that can be disconnected from the apparatus). The removable display may be configured to connect to the apparatus (for example the controller of the apparatus) via a wired or wireless connection to transmit and receive data from the apparatus (for example the controller of the apparatus).

The display module may be a remote display located external to the apparatus.

The display module may be configured to receive inputs to the system (for example as a touch screen).

The display module may be configured to be an input/output (I/O) module. For example, the display module may be configured to receive inputs from a user and provide outputs to a user.

The display module may communicate with the controller 13. In some embodiments the display module may provide information to the controller 13 (for example set points). In some embodiments the display module may receive information from the controller 13 (for example alarms, sensor outputs, and/or other calculated variables.)

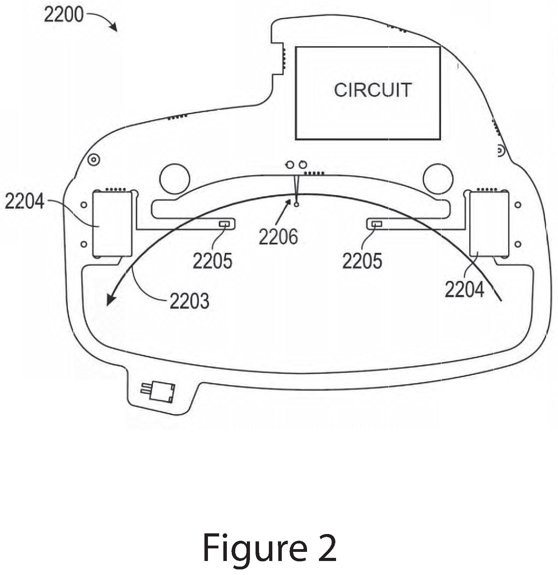

With additional reference to Figure 2, a sensing circuit board 2200 is shown that can be implemented in the breathing assistance apparatus 10. The sensing circuit board 2200 can be positioned in a sensor chamber such that the sensing circuit board 2200 is at least partially immersed in the flow of gases. The flow of gases may exit the blower 11 through a conduit and enter a flow path in the sensor chamber. At least some of the sensors on the sensing circuit board 2200 can be positioned within the flow of gases (shown in direction by arrow 2203) to measure gas properties within the flow. After passing through the flow path in the sensor chamber, the gases can exit to the humidifier 12 described above.

The sensing circuit board 2200 can be a printed sensing circuit board (PCB). Alternatively, the circuit on the board 2200 can be built with electrical wires connecting the electronic components instead of being printed on a circuit board. At least a portion of the sensing circuit board 2200 can be mounted outside of a flow of gases. The flow of gases can be generated by the flow generator 11 described above. The sensing circuit board 2200 can comprise ultrasonic transducers 2204. The sensing circuit board 2200 can comprise one or more thermistors 2205. The thermistors 2205 can be configured to measure a temperature of the gases flow. The sensing circuit board 2200 can comprise a thermistor flow rate sensor 2206. The sensing circuit board 2200 can comprise other types of sensors, such as humidity sensors (including humidity only sensors to be used with a separate temperature sensor and combined humidity and temperature sensors), sensors for measuring barometric pressure, sensors for measuring differential pressure, and/or sensors for measuring gauge pressure. The thermistor flow rate sensor 2206 can comprise a hot wire anemometer, such as a platinum wire, and/or a thermistor, such as a negative temperature coefficient (NTC) or positive temperature coefficient (PTC) thermistor. Other non-limiting examples of the heated temperature sensing element include glass or epoxy-encapsulated or non-encapsulated thermistors. The thermistor flow rate sensor 2206 can be configured to measure the flow rate of the gases by being supplied with a constant power, or by being maintained at a constant temperature or a constant temperature difference between the sensor and the flow of gases.

Positioning the one or more of thermistors 2205 and/or the thermistor flow rate sensor 2206 downstream of the combined blower and mixer means that the sensor readings will be dependent on the heat supplied to the gases flow by the blower. Furthermore, immersing at least part of the sensing circuit board and sensors in the flow path can increase the accuracy of measurements. Relative to sensors that are not immersed, sensors that are immersed in the flow are more likely to be subject to the same conditions, such as temperature and pressure, as the gases flow. Therefore, these immersed sensors may provide a better representation of the gases flow characteristics.

The sensing circuit board 2200 can comprise ultrasonic transducers, transceivers, or other sensors to measure properties of the gases flow, such as gas composition or concentration of one or more gases within the gases stream. Any suitable transducer, transceiver, or sensor may be mounted to the sensing circuit board 2200 as will be appreciated. In this configuration, the gas composition sensor is an ultrasonic transducer that employs ultrasonic or acoustic waves for determining gas concentrations.

Some examples of flow therapy apparatuses are disclosed in International Application No. PCT/NZ2016/050193, titled "Flow Path Sensing for Flow Therapy Apparatus", filed on December 2, 2016, and International Application No. PCT/IB2016/053761, titled "Breathing Assistance Apparatus", filed on June 24, 2016, which are hereby incorporated by reference in their entireties.

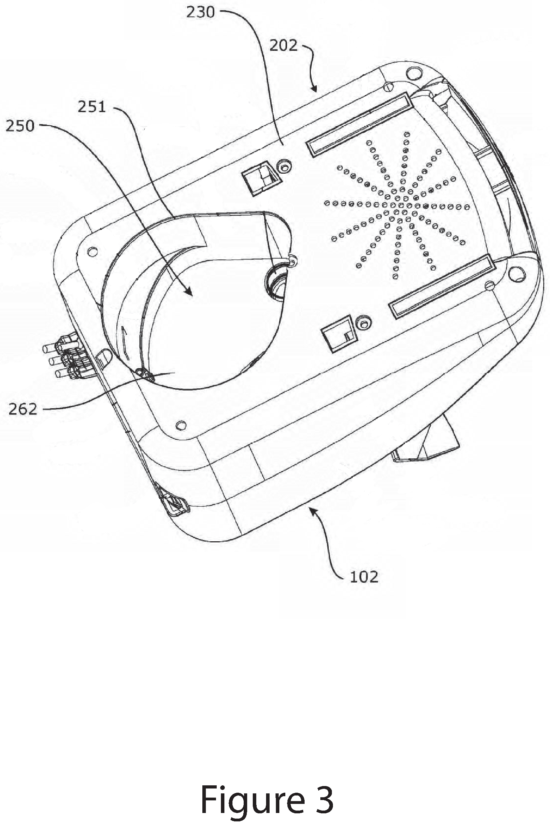

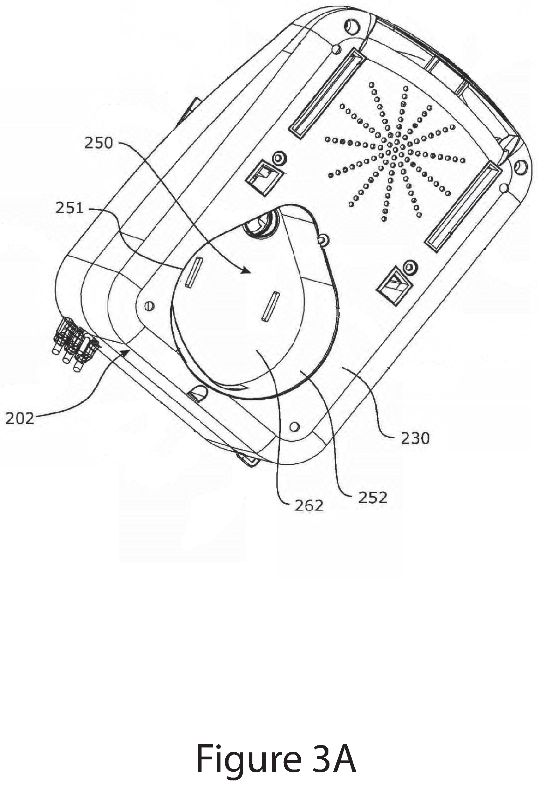

A configuration of a breathing assistance apparatus 10 is illustrated in Figures 3 to 7. As shown for example in Figure 4, the breathing assistance apparatus comprises a main housing 100. The main housing 100 has a main housing upper chassis 102 and a main housing lower chassis 202.

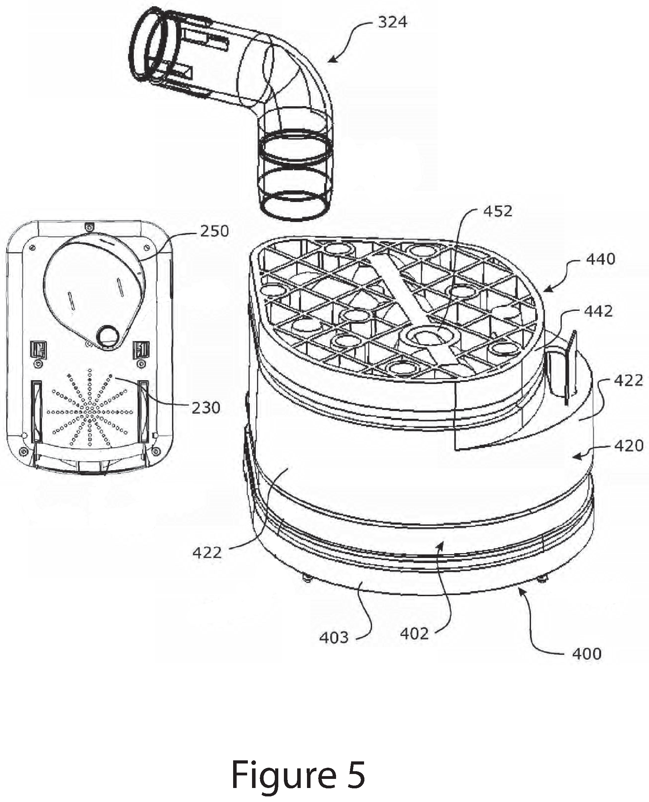

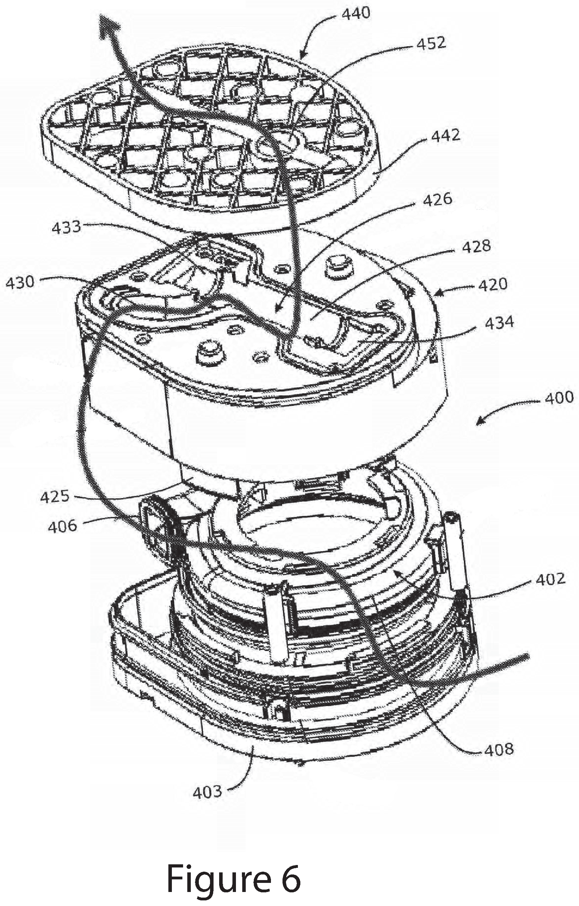

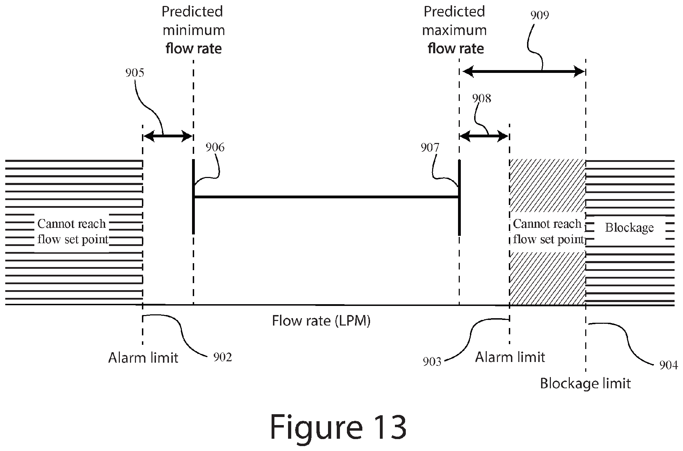

As shown in figures 3 and 3A, the lower chassis 202 has a motor recess 250 for receipt of a removable or non-removable motor and/or sensor module 400 which is shown in figures 13 to 15 and will be described in further detail below. A recess opening 251 is provided in the bottom wall 230 adjacent a rear edge thereof, for receipt of a removable or non-removable motor/sensor module 400 which is shown in figures 5 and 6 and will be described in further detail below.

Figures 5 to 7 show the motor and/or sensor module or sub-assembly 400 in greater detail. As discussed above, the lower chassis 202 comprises a recess 250 for receipt of the motor and/or sensor module 400.

In the form shown in figures 5 to 7, the motor and/or sensor module 400 comprises a stacked arrangement of three main components: a base 403 of the sub-assembly 400 (on which is positioned the motor 402), an outlet gas flow path and sensing layer 420 positioned above the base 403, and a cover layer 440. The base 403, the sensing layer 420, and the cover layer 440 assemble to form a sub-assembly housing that has a shape that is complementary to that of the recess 250 so that the sub-assembly 400 can be received in the recess 250. The base 403 is configured to close the recess opening 251 when the sub-assembly 400 is positioned in the recess 250. The sub-assembly 400 may be maintained in position in the recess in any suitable way such as with fasteners, clips, or a quick release arrangement for example, or fixed in a non-removable manner.

The sensing layer comprises a gas flow path with one or more sensors, the gas flow path arranged to deliver gas to the outlet port of the housing.

The motor 402 has a body 408 that defines an impeller chamber that contains an impeller. The motor 402 could be any suitable gas blower motor, and may for example be a motor and impeller assembly of the type described in published PCT specification WO2013/009193. The contents of that specification are incorporated herein in their entirety by way of reference.

A gases outlet 406 is in fluid communication with a gases inlet of the outlet gas flow path and sensing layer 420, which is stacked on top of the motor. This layer 420 comprises a body 422 which comprises a plurality of mounting legs 425 that can be inserted into a plurality of mounting slots (not shown) of the base 403 to secure the body 422 to the base 403. In one configuration, the body 422 defines a gas flow path that couples the gases outlet 406 with the gases inlet of the gas flow path and sensing layer 420.

The body 422 defines a lower portion 426 of a sensing and gas flow path. The cover layer 440 has a body 442 that defines the upper portion 446 of the sensing and gas flow path, with the shape of the upper and lower portions 426, 446 corresponding substantially to each other.

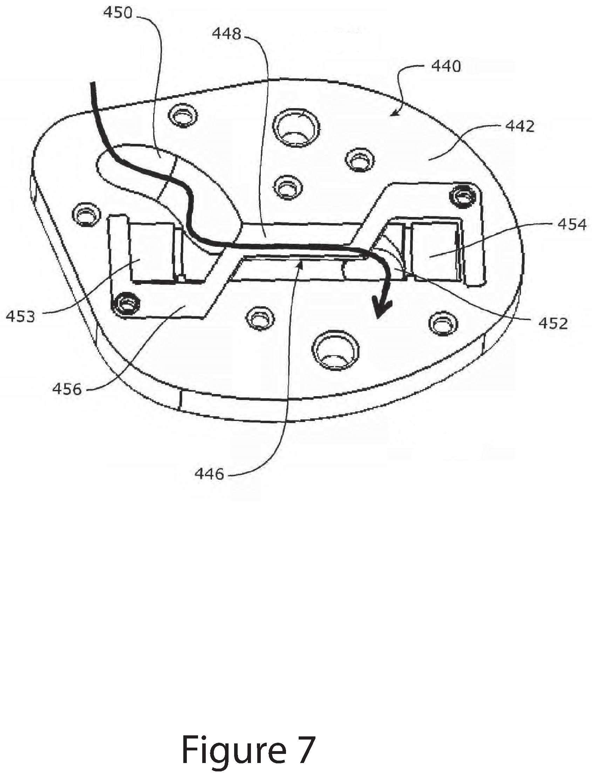

As shown in figures 6 and 7, the gas flow path comprises a linear elongate gas flow portion 428, 448. The inlet is in fluid communication with a tangential entrance portion 430, 450 of the gas flow path, which is located at or adjacent an entrance end of the linear elongate portion 428, 448 of the gas flow path. Recesses 433, 453 and 434, 454 may be provided at opposite ends of the linear elongate portion of the gas flow path.

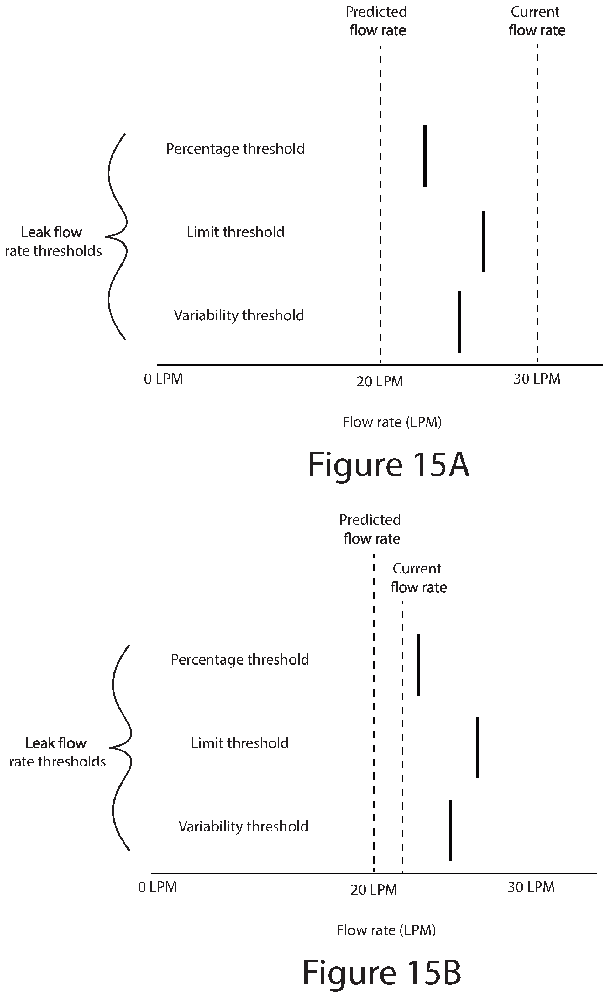

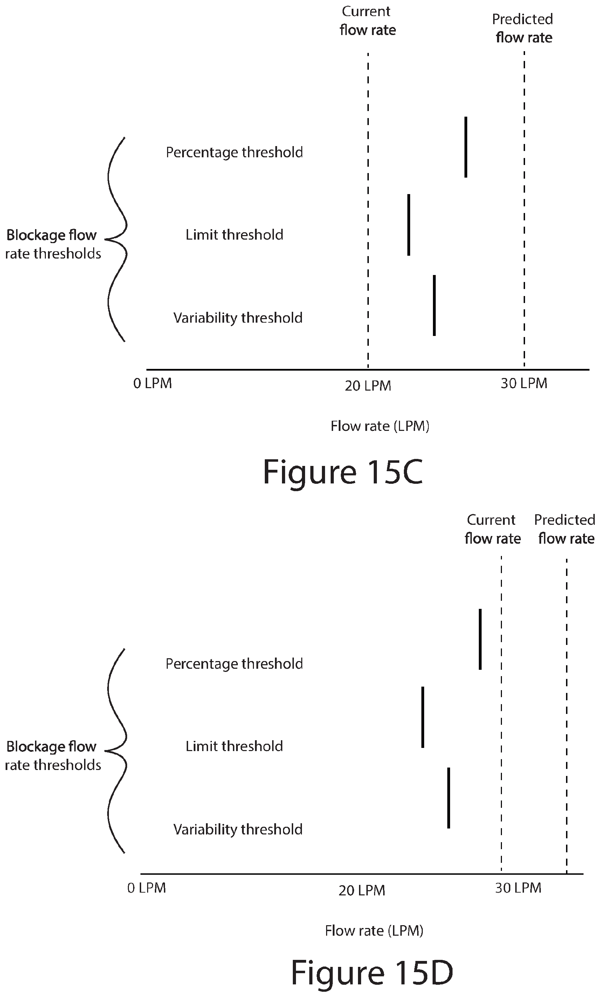

A gas flow outlet port 452 extends vertically through the body 442 of the cover layer 440 and is located at or adjacent an opposite exit end of the linear elongate portion 428, 448 of the gas flow path. The gas outlet port 452 is in fluid communication with an upper portion of the motor recess 250, which in turn is in fluid communication with the gas flow passage. Again, due to the wall 252 and ceiling 262 configuration of the recess 250, if there is gas leakage from the motor/sensor module 400, that will be vented to atmosphere rather than entering the portion of the main housing 100 that contains the bulk of the electronics and control equipment. The recess 250 may comprise spacer(s), such as lugs that protrude downwardly from ceiling 262 as shown in figure 15, to maintain a suitable spacing for gas flow from the gas outlet port 452 and the ceiling of the recess 262.

It can be seen from figure 7 that that at least part of the gas flow path through and out of the motor and/or sensing module 400 has a tortuous or sinuous configuration. For example, the direction of gas flow travel through the elongate portions 428, 448 is generally opposite to the direction of gas flow travel from the gas outlet port 452 to the entrance of the gas flow passage through elbow 324.

As shown in figures 6 and 7, the cover layer 440 comprises a sensing printed circuit board (PCB) 456. The cover layer 440 may also comprise one or more temperature sensors such as thermistors that sit in the elongate portion 428, 448 of the gas flow path. One sensor will measure gas temperature and the other can act as a redundant temperature sensor. Alternatively, one of the thermistors could be used as a reference flow sensor (e.g. via use as a constant-temperature thermistor), and the measured temperatures could be used to determine the gas flow rate through the portion 428, 448 of the gas flow path. The one or more temperature sensors may be located on a portion of the sensing PCB 456 that faces the gases flow. The sensing PCB 456 may additionally comprise other sensors including but not limited to pressure sensors, humidity sensors, and dew point sensors.

One or both electronics boards 272 will be in electrical communication or coupled with the sensors to process information received from the sensors and operate the apparatus 10 based on the information received from the sensors.

In an alternative configuration, the motor/impeller unit may be provided remotely from the apparatus 10. In that configuration, the module received in the recess 250 may only comprise a gas flow path and various sensors, to deliver gases to the fixed elbow 324 and thereby to the liquid chamber 300. In an alternative configuration, the module received in the recess 250 may only comprise the motor and a gas flow path, but no sensors.

In another alternative configuration the motor and/or sensor module 400 may not be removable from the recess 250, but instead may be permanently mounted therein.

Some configurations may provide the benefit of isolating the electrical/electronic components from the gases in the gases flow.

The flow path is compact and has reduced turns/sharp turns which reduces flow separation and reduces resistance to flow.

The arrangement of the motor and flow path provides another layer of isolation because of the wall arrangement.

Having a modular motor and/or sensor module enables the various parts of the module to be taken apart if needed for cleaning and/or servicing.

In some embodiments, there are no leak paths in the motor and/or sensor module.

In some embodiments, the motor and/or sensor module may be a potential leak point, however, a leak in that region would result in the oxygen venting to atmosphere or into the liquid chamber.

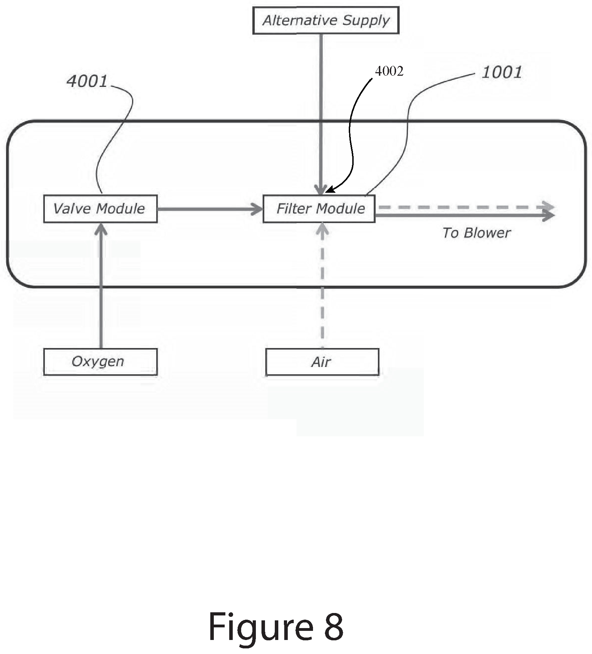

For example, as shown in Figure 8, the apparatus 10 may comprise a valve module that controls the flow of oxygen and/or other gases entering the gas flow path of the apparatus 10, and enables the apparatus 10 to regulate the proportion of oxygen entrained in the airflow. The valve module is formed as a modular unit for ease of manufacture, assembly, servicing, or replacement. For example, in the event of malfunction, routine maintenance, or future upgrade/improvement.

The apparatus 10 may comprise a filter module 1001, which may comprise a filter.

The filter modules and valve modules described herein may provide varying gas flow paths for the apparatus. For example, the valve module may control the flow of oxygen entering the gas flow path of the apparatus, via the valve module and filter module. Alternatively, the valve module may be bypassed by means of direct connection of an alternative oxygen source to the filter module via an alternative supply inlet. This may be practical in circumstances where a user may wish to manually adjust the oxygen supply (i.e. by a wall-supply rotameter).

It will be appreciated that the filter modules and the valve modules described herein may be used separately in apparatuses for delivering a flow of gas. Alternatively, the filter and the valve module may be used together as a filter and valve assembly for improved functionality.

In the configurations shown, the apparatus 10 receives oxygen by at least one of the following:
via the valve module (for automatic oxygen regulation by the apparatus), or
via the alternative gases inlet provided on the top of the filter (allowing attachment of a manually adjustable oxygen supply - such as a wall supply regulated by a regulator).

The various configurations described are exemplary configurations only. Any one or more features from any of the configurations may be used in combination with any one or more features from any of the other configurations.

As another example, while the motor and/or sensor sub-assembly recess is described as being in the underside of the main housing, it could alternatively be in the rear, side, front, or top of the housing. The air and/or oxygen inlets may also be positioned differently as required.

As another example, rather than the liquid chamber and chamber bay being configured so that the liquid chamber is inserted into and removed from the chamber bay from a front of the housing, the configuration could be such that the liquid chamber is inserted into and removed from the chamber bay from a side, rear, or top of the housing.

As another example, while the filter modules are described as being inserted into the housing from above and the valve modules inserted into the housing from below, either or both of those components could be inserted into any suitable part of the housing, such as an upper part, lower part, side part, front part, or rear part.

The filter module and valve module are described with reference to a breathing assistance apparatus that can deliver heated and humidified gases to a patient or user. The apparatus may be suitable for treating patients with chronic obstructive pulmonary disease (COPD) and/or patients that are experiencing respiratory compromise. For example, treating patients with dyspnea, low Oxygen saturation (i.e. SpO2), or high partial pressure of carbon dioxide (I.e. PaCO2). The apparatus may be configured to deliver gases to a patient interface at a high flow rate (i.e. high flow therapy - particularly nasal high flow therapy). Nasal high flow therapy can also be used in treating infections that cause respiratory distress e.g. COVID 19.

Alternatively, the filter module and/or valve module may be used in an apparatus for a different purpose. The apparatus may be a high flow therapy apparatus, or may be a low flow therapy apparatus. The features may also be provided in an apparatus for providing continuous positive airway pressure (CPAP), which may deliver gases (humidified or otherwise) at positive pressure.

The filter module and/or valve module may alternatively be used with an apparatus that does not require a humidifier and therefore does not require the liquid chamber 300. For example, it will be appreciated that the configuration that isolates the motor and gas flow path from the electrical and electronic components has broad applications in other types of gas delivery apparatuses.

Embodiments will now be described relating to methods and apparatus for predicting minimum and/or maximum flow rates provided by a breathing apparatus.

As described above, a user or clinician may enter a flow rate set point to the apparatus via a user interface. The apparatus will then attempt to control a motor or other flow generator to provide a flow rate at the flow rate set point.

If the flow rate cannot be delivered to a patient at the flow rate set point with the particular flow path (for example if the conductance of the flow path is too high or too low) the patient may not receive adequate therapy.

For example, if the flow rate set point is greater than a predicted maximum flow rate for the flow path, the patient may receive a flow rate (for example, a flow rate equal to the predicted maximum flow rate for the flow path) which is less than that anticipated by a clinician. This is because for the current conditions of the flow path, the apparatus is unable to provide a high enough flow rate to meet the flow rate set point (which may be indicative of a blockage, for example).

In another example, if the flow rate set point is less than a minimum predicted flow rate, the user may receive a flow rate which is higher than anticipated by a clinician, as, for the current conditions of the flow path, the apparatus is unable to provide a low enough flow rate to meet the flow rate set point. In this case the patient may not be receiving adequate therapy which may present a risk to the patient.

Prior art systems may only provide alarms when the flow rate of the breathing gases does not reach a flow rate set point for a period of time. However, in some cases, some of these types of systems may take a long time to detect such a condition and generate an alarm, and therefore there can be a delay before a clinician or user is notified. During this time the patient may not be receiving a flow rate at the flow rate set point, and therefore may not be receiving adequate therapy.

Determining the predicted maximum and minimum flow rates for the particular flow path (based on the allowable flow generator output range and the conductance of the flow path) may significantly reduce the time for which inadequate therapy (for example, therapy in which the flow rate set point cannot be reached) is provided. Determining the predicted maximum and minimum flow rates for the particular flow path may also reduce the time taken to generate an alarm (and, for example, the time for a clinician to react and take any necessary action).

Further, as the apparatus 10 does not have to try to reach the flow rate set point to determine the flow rate set point cannot be reached, the time taken to generate an alarm (that indicates that the flow rate set point cannot be reached) may be reduced.

The predicted minimum and/or maximum flow rates may be based on a variable indicative of conductance of the flow path through which the breathing gases are provided, and an allowable flow generator output range. For example, once the variable indicative of conductance is calculated, the maximum and minimum flow rates may be predicted as a function of this variable indicative of conductance and the maximum and minimum flow generator outputs respectively. These predicted maximum and minimum flow rates represent the bounds of the predicted range of flow rates that the flow generator could provide, given the range of allowable flow generator outputs.

The variable indicative of conductance of the flow path may be the conductance of the flow path or based on the conductance of the flow path (for example based on conductance data as described in more detail below).

The apparatus may additionally or alternatively determine, based on the minimum and maximum flow rate, whether the apparatus is or is not going to be able to achieve the flow rate set point (given the current flow path conditions).

As described above, for example, the minimum and maximum flow rates are based on a variable indicative of conductance of the flow path.

The flow path may comprise one or more components. The components which make up the flow path may be removable and/or interchangeable and each component may have one or more alternatives. For example, there may be a number of possible patient interfaces which can be connected to form part of the flow path.

The flow path may comprise one or more of: a humidification chamber, one or more breathing conduits, a patient interface, an elbow, and a sensor module.

The flow path may be downstream of the flow generator 10 (and optionally the output of the flow generator 10).

The flow path may be downstream of the humidifier 12 (and optionally the output of the humification chamber).

The flow path may be downstream of the elbow.

The flow path may be downstream of the sensor module.

Depending on for example sensor locations, the flow path (for which the variable indicative of conductance is determined) may be defined differently. For example, if a sensor is used which is located at the output of the humidifier (i.e. 3b) or the inlet of the conduit (i.e. 3c) the flow path may be defined as downstream from this sensor.

In some embodiments, the flow path may comprise at least a humidification chamber, a patient interface and a conduit (and optionally a sensor module).

In some embodiments, the flow path may comprise at least a patient interface and a conduit (and optionally a sensor module).

The value or values of the variable indicative of conductance of the flow path will depend on the particular components within it. For example, the variable indicative of conductance for a flow path with a larger diameter conduit may be greater than the variable indicative of conductance for the same flow path with a smaller diameter conduit.

The variable indicative of conductance of the flow path may be based on parameters external to the flow path, for example the patient's anatomy or condition, and the connection between the patient and the patient interface.

The variable indicative of conductance of the flow path may also be based on characteristics of the connection between the patient interface 17 with the patient. For example, a tighter fit between the prongs and the nares may lead to a lower variable indicative of conductance for the flow path (as less of the flow of gases would be exhausted to atmosphere). However, if the fit between the prongs that the nares is looser, more of the flow of gases would be exhausted to atmosphere and therefore the variable indicative of conductance for the flow path may be greater. This effect would be more pronounced in non-sealing systems as a significant portion of the variable indicative of conductance would be based on the connection between the patient interface 17 with the patient.

As a further example, deformation of the nasal prongs may also influence the variable indicative of conductance of path.

The variable indicative of conductance of path may also be based on characteristics of the patient's respiratory system. For example, structural characteristics of the patient's respiratory system may lead to differences in conductance of the flow path.

The variable indicative of conductance of the flow path defines a relationship between the flow generator output and a flow rate of the breathing gases.

The variable indicative of conductance of the flow path may be or comprise: a conductance of the flow path (or a portion of the flow path); a resistance to flow of the flow path (or a portion of the flow path); or a pressure drop along the flow path (or a portion of the flow path), or any combination of the above.

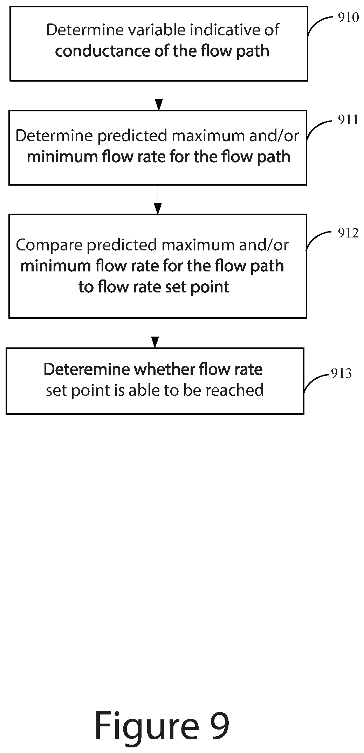

Figure 9 shows an example of the process for determining whether the flow rate set point is able to be reached.

At step 910, the controller 13 determines a variable indicative of conductance of the flow path.

At step 911, the controller 13 determines a maximum and/or minimum flow rate for the flow path.

At step 912, the controller 13 compares the maximum and/or minimum flow rate for the flow path to the flow rate set point.

At step 912, the controller 13 determines whether the flow rate set point is able to be reached.

As described above the apparatus may comprise a controller 13. The controller 13 is configured to control a flow rate of the breathable gas.

The controller 13 may control the flow rate of the breathable gas by controlling a flow generator output (for example, motor speed).

The controller 13 may generate said flow generator output based on one or more control algorithms.

The controller 13 may be configured to control the flow generator output within an allowable flow generator output range. The allowable flow generator output range may indicate operating limits for which the flow generator can safely operate. In some embodiments the allowable flow generator output range may indicate safety limits.

In some embodiments, for example where the flow generator comprises a motor, the flow generator output may be a motor output. Herein, the flow generator output is described in relation to a motor output, however it will be appreciated that depending on the type of flow generator the flow generator output may be any output. The flow generator output might in some embodiments be, for example, a target pressure for the flow path.

The allowable motor output range (for example, the flow generator output range, as described above) comprises an upper motor speed limit and/or a lower motor speed limit.

The allowable motor output range may be based on the flow rate set point. For example, the magnitude and/or the limits of the allowable motor output range may be proportional, or otherwise mathematically related to the flow rate set point.

The predicted maximum flow rate is based on an upper motor speed limit. The predicted minimum flow rate may be based on a lower motor speed limit.

In some embodiments the predicted maximum flow may be additionally based on the current motor speed.

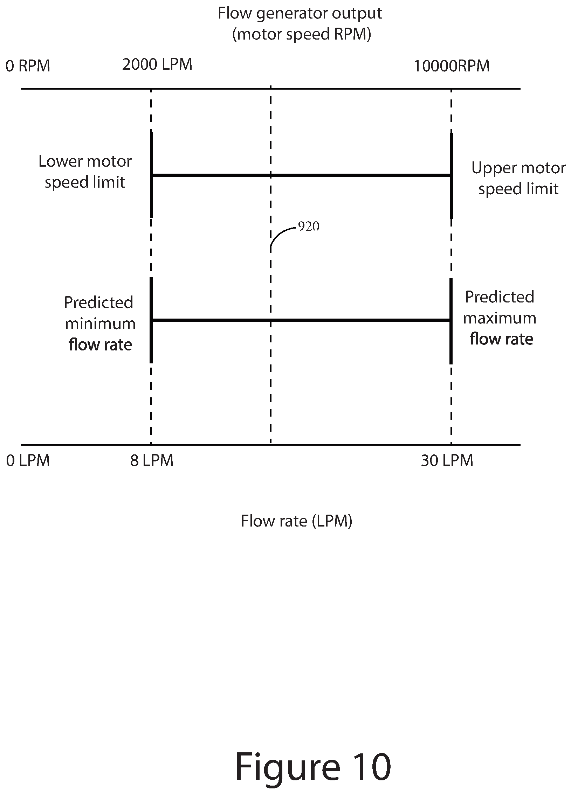

Figure 10 illustrates an example of the relationship between the motor speed limits and the predicted flow rates. For example, the predicted minimum flow rate as shown in Figure 10 (8 Litres per minute (LPM)) corresponds with the lower motor speed limit (2,000RPM), and the predicted maximum flow rate (30LPM) corresponds with the upper motor speed limit (10,000RPM). In this example, the relationship between the motor speed and the predicted flow rate is based on the variable indicative of conductance of the flow path.

Reference 920 of Figure 10 indicates an example of a flow rate and motor speed relationship within the allowable motor speed range. It will be appreciated that the variable indicative of conductance of the flow path may represent or be based on the relationship between flow rate and motor speed for the flow path.

In some embodiments, the predicted maximum flow rate may be determined by evaluating the difference between the current motor speed and an upper motor speed limit. Similarly, the predicted minimum flow rate may be based on a difference between the current motor speed and a lower motor speed limit.

The motor output 1306 may be or comprise: a motor speed, a motor current, a motor duty (duty cycle), a pressure of the gases in the flow path, or any combination of the above.

The controller 13 is configured to determine a variable indicative of conductance of the flow path.

The variable indicative of conductance of the flow path may be based on a variable indicative of conductance of the flow path at a previous time step.

The variable indicative of conductance of the flow path may be based on another variable indicative of conductance of the flow path over a timing window, optionally the timing window is a preceding time period for example the preceding 20 seconds. For example, the variable indicative of conductance may be based on a number of time weighted average conductance of the flow path.

The variable indicative of conductance of the flow path may be a function of the current motor output and the current flow rate.

In one instance of the present invention, the variable indicative of conductance of the flow path can be proportional to the current flow rate divided by the current motor output. For example: $\frac{flow rate}{motor output}$

The use of motor speed in determining the variable indicative of conductance may be of particular benefit in a non-sealed system, as other variables which may be used in determining conductance (for example pressure) may be difficult to use due to the unsealed patient interface.

In another instance, the variable indicative of conductance of the flow path can be proportional to the current flow divided by the square root of the current pressure. For example: $\frac{flow rate}{\sqrt{pressure}} .$

The predicted maximum flow rate and/or the predicted minimum flow may be determined periodically (optionally at each time step of the controller 13).

The controller 13 may also be configured to compare the predicted maximum flow rate and/or the predicted minimum flow rate to one or more flow rate set points.

Based on the comparison of the predicted maximum flow rate and/or the predicted minimum flow rate to one or more flow rate set points, the controller may further be configured to generate or activate at least one alarm condition.

The at least one alarm condition may comprise, a cannot reach flow rate set point alarm, and/or a blockage alarm.

The alarm condition in relation to the cannot reach flow rate set point may comprise a cannot reach flow rate alarm associated with the flow rate set point being above the maximum predicted flow rate.

The alarm condition in relation to the cannot reach flow rate set point may comprise a cannot reach flow rate alarm associated with the flow rate set point being below the minimum predicted flow rate.

Additionally, or alternatively, to the alarms as described in the specification, the alarms may show a generic alarm indication (for example a message which may be explained in an associated manual) and/or an indication that prompts a user to check for a specific fault with the system that might cause the alarm. The description above with respect to alarms is an example and it will be appreciated that specific wording may be modified.

In some conditions, the lower flow generator output limit (for example a lower motor output limit) may prevent the apparatus from reaching the flow rate set point. For example, where the apparatus is prevented from reducing the flow generator output below the lower flow generator output limit. Such conditions could occur if, for example, one or more components are disconnected from the flow path, one or more components are improperly connected, one or more components are improperly used, and/or one or more incorrect components are used.

The controller 13 may be configured to determine, based on the predicted minimum flow rate and the flow rate set point, a condition where a flow rate set point cannot be reached, and in response generate a cannot reach flow rate set point alarm.

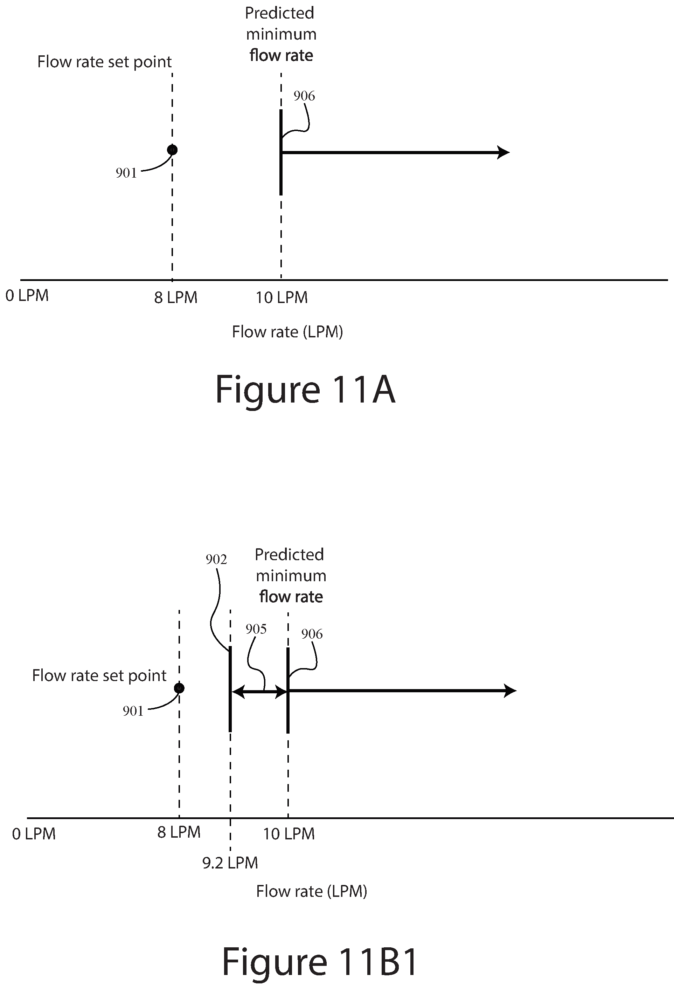

In some embodiments the controller 13 may be configured to generate at least one alarm condition when the flow rate set point is less than the predicted minimum flow rate (optionally the alarm condition is a cannot reach flow rate set point alarm). An example of this condition is shown in Figure 11A. In Figure 11A, the flow rate set point 901 is 8 LPM which is below the predicted maximum flow rate (10 LPM).

Note that Figures 11A - 11B2, and 12A - 12D show example portions of the flow range illustrated in Figure 13.

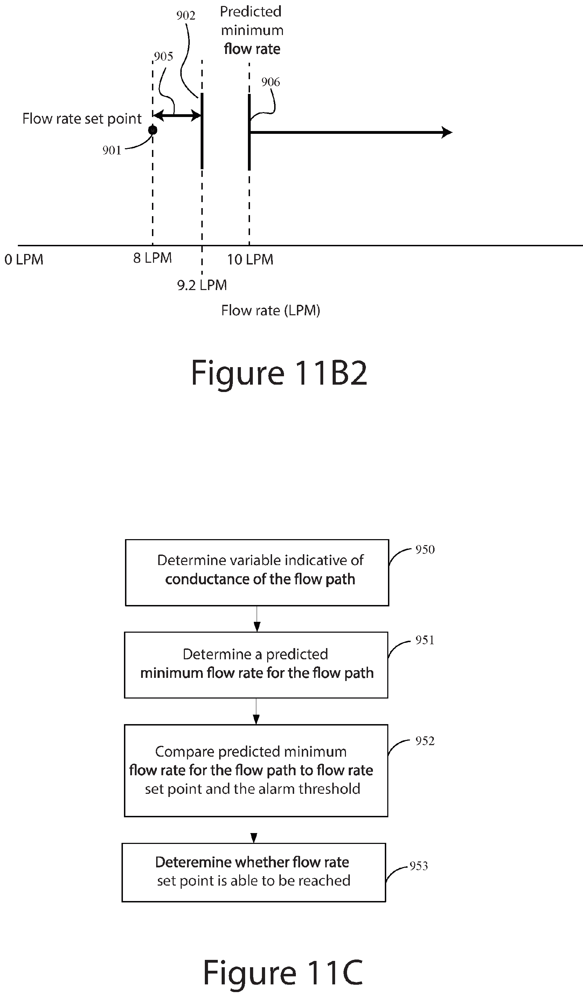

In some embodiments the controller 13 may be configured to generate at least one alarm condition when the flow rate set point is less than the predicted minimum flow rate by more than an alarm threshold. In Figure 11B1, the flow rate set point 901 is 8 LPM which is below the predicted minimum flow rate 906 (10 LPM) by more than an alarm threshold 905 (0.8 LPM). For example, if the flow rate set point was less than 9.2 LPM then the alarm condition would be raised.

The predicted minimum flow rate minus the alarm threshold 905 may define an alarm limit 902.

In some embodiments the controller 13 may be configured to generate at least one alarm condition when the predicted minimum flow rate is greater than a flow rate set point by more than an alarm threshold. In Figure 11B2, the predicted minimum flow rate 906 is greater than the flow rate set point 901 (8 LPM) by more than an alarm threshold 905 (1.2 LPM). For example, if the predicted minimum flow rate was greater than 9.2 LPM then the alarm condition would be raised.

The alarm threshold is a proportion of the flow rate set point. The alarm threshold may be about 5% to about 20% or about 10% of the flow rate set point. Figure 11B shows an example of this condition where the alarm threshold 905 is 0.8 LPM (i.e. 10% of the flow rate set point - 8 LPM).

The alarm condition may be a "cannot reach flow rate set point" alarm, and optionally, a "cannot reach flow rate set point - set point below minimum flow" alarm.

In some embodiments, the alarm threshold may be a proportion of the predicted minimum flow rate. In some embodiments the alarm threshold may be a set amount.

Figure 11C shows an example of the process for determining whether a flow rate set point is able to be reached. If the flow rate set point cannot be reached, a cannot reach flow rate set point alarm may be generated.

At step 950, the controller 13 determines the variable indicative of conductance (as described in more detail elsewhere in the specification). The controller 13 within the breathing apparatus may perform step 950 at each time step during therapy.

At step 951, the predicted minimum flow rate is determined based on the variable indicative of conductance (calculated at step 950) and the lowest motor output (which in this example represents flow generator output) of the allowable motor output range.

At step 952, the predicted minimum flow is compared to the flow rate set point and an alarm threshold (as shown in Figure 11B, for example).

At step 953, the controller 13 determines, based on the comparison at step 952, whether the flow rate set point can be reached. If the flow rate set point is less than the predicted minimum flow rate by more than an alarm threshold, the controller 13 may generate an alarm condition (i.e. a "cannot-reach-set-flow" alarm).

In some embodiments, the controller 13 may require a number of instances of the flow rate set point being less than the predicted minimum flow rate by more than an alarm threshold. In these embodiments the controller 13 may require a number of consecutive "cannot-reach-set-flow" events to exceed a consecutive-count threshold. If the consecutive-count threshold is exceeded, the controller 13 may then generate an alarm condition indicating the flow rate set point is less than the predicted minimum flow rate (optionally the alarm condition is a cannot reach flow rate set point alarm).

In some embodiments, the controller 13 may require a threshold time to generate an alarm condition. For example, the controller 13 may require a threshold time for which the flow rate set point is less than the predicted minimum flow rate by more than an alarm threshold. In these embodiments the controller 13 may require the time for which a "cannot-reach-set-flow" event is active to exceed threshold time. If the threshold time is exceeded, the controller 13 may then generate an alarm condition indicating the flow rate set point is less than the predicted minimum flow rate (optionally the alarm condition is a cannot reach flow rate set point alarm). In some embodiments, the threshold time may be about 1 second to about 5 seconds.

In some conditions the upper flow generator limit (for example an upper motor speed limit) may prevent the apparatus from reaching the flow rate set point. For example, where the apparatus is prevented from increasing the flow generator output above the upper flow generator output limit. This may be because, for example, the flow rate set point entered by the user is higher than appropriate for the patient interface, the patient interface is too large for the patient, there is an occlusion or a kink in the tube, or there is some other blockage in the flow path (caused by a patient laying on the tube for example).

The controller 13 may be configured to determine, based on the predicted maximum flow rate and the flow rate set point, a condition where a flow rate set point cannot be reached, and in response, generate a cannot reach flow rate set point alarm.

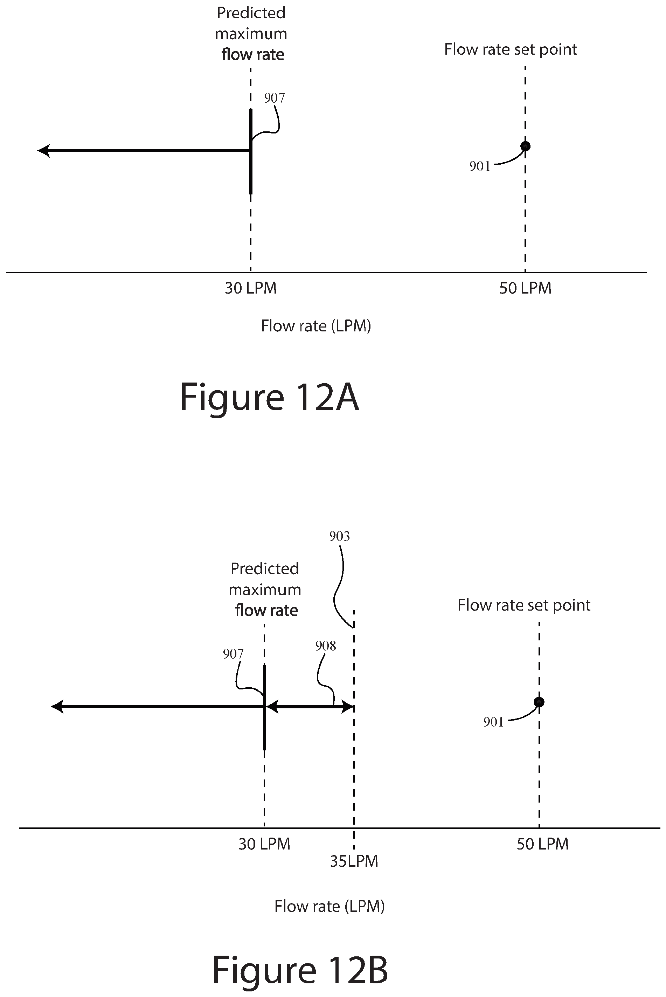

In some embodiments the controller 13 may be configured to generate at least one alarm condition when the flow rate set point is greater than the predicted maximum flow rate (optionally the alarm condition is a cannot reach flow rate set point alarm). In Figure 12A, the flow rate set point 901 is 40 LPM which is greater than the predicted maximum flow rate 906 (30 LPM).

In some embodiments the controller 13 may be configured to generate at least one alarm condition when the flow rate set point is greater than the predicted maximum flow rate by more than an alarm threshold.

In some embodiments the controller 13 may be configured to generate at least one alarm condition when the predicted maximum flow rate is less that the flow rate set point by more than an alarm threshold 908.

The predicted maximum flow rate plus the alarm threshold 908 may define an alarm limit 903.

The alarm threshold is a proportion of the flow rate set point. The alarm threshold may be about 5% to about 20% or about 10% of the flow rate set point. Figure 12B shows an example of this condition where the alarm threshold is 5 LPM (i.e. 10% of the flow rate set point - 50 LPM).

The alarm condition may be a cannot reach flow rate set point" alarm, and optionally, a "cannot reach flow rate set point - set point too high" alarm.

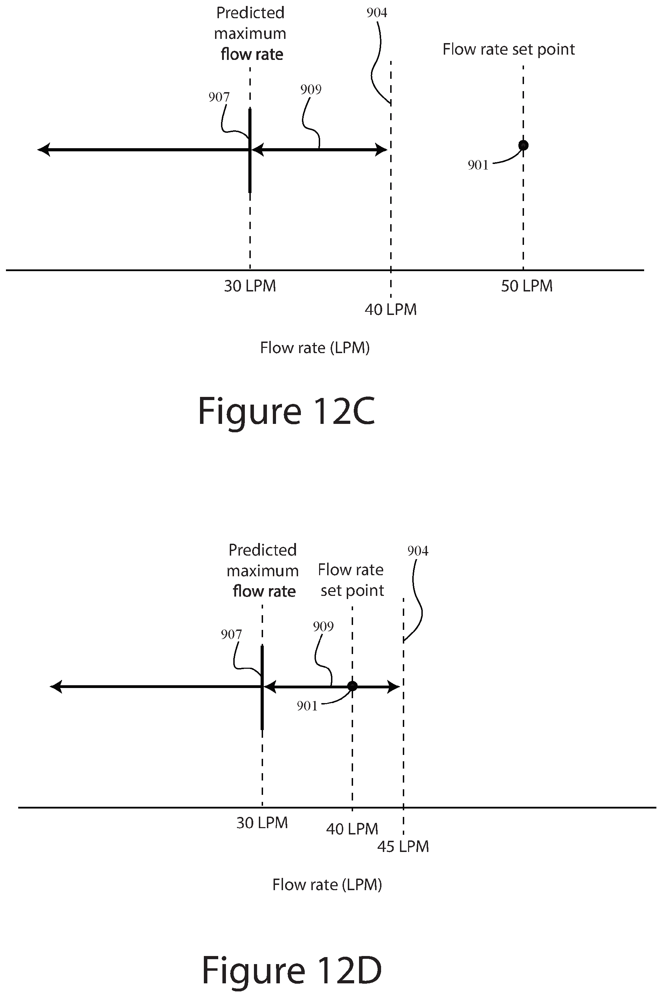

In some embodiments, if the flow rate set point is greater than the predicted maximum flow rate by more than a blockage threshold, the controller is configured to generate a blockage alarm. For example, in Figure 12C the flow rate set point 901 is 50 LPM which is above the predicted maximum flow rate 907 (30 LPM) by more than a blockage threshold 909 (10LPM).

The blockage threshold may be indicative of a large enough difference between the flow rate set point and the predicted maximum flow rate (for example where the flow rate set point is much higher than the predicted maximum flow rate) to be indicative of a blockage in the flow path.

The predicted maximum flow rate plus the blockage threshold may define a blockage limit 904.

In some embodiments, if the predicted maximum flow rate is less than a flow rate set point by more than a blockage threshold the controller is configured to generate a blockage alarm. For example, in Figure 12C the predicted maximum flow rate 907 (30 LPM) is less than a flow rate set point 901 (50 LPM) by more than a blockage threshold 909 (10 LPM).

The blockage threshold is about 20% to about 70%, or about 50% of the flow rate set point. Figure 12C shows an example of this condition where the blockage threshold is 10 LPM (i.e. 20% of the flow rate set point - 50 LPM). Figure 12D shows an example of this condition where the blockage threshold is 15 LPM (i.e. 37.5% of the flow rate set point - 40 LPM).

In some embodiments the blockage threshold may be a proportion of the predicted maximum flow rate, or, for example, a set amount.

In some embodiments, the proportion of the predicted maximum flow rate defining the blockage threshold may be based on the predicted maximum flow rate.

The alarm condition may be a blockage alarm.

It will be appreciated that the cannot reach flow rate set point alarm and blockage alarm may be determined and raised independently. For example, as shown in Figure 12D, where the flow rate set point is greater than the predicted maximum flow rate, but not greater than the predicted maximum flow rate by more than the blockage threshold and so the cannot reach flow rate set point alarm may be raised, but the blockage alarm will not.

In some embodiments, for example as shown in Figure 13 both the alarm limit 903 and blockage limit 904 may be both utilised at the same time as described above.

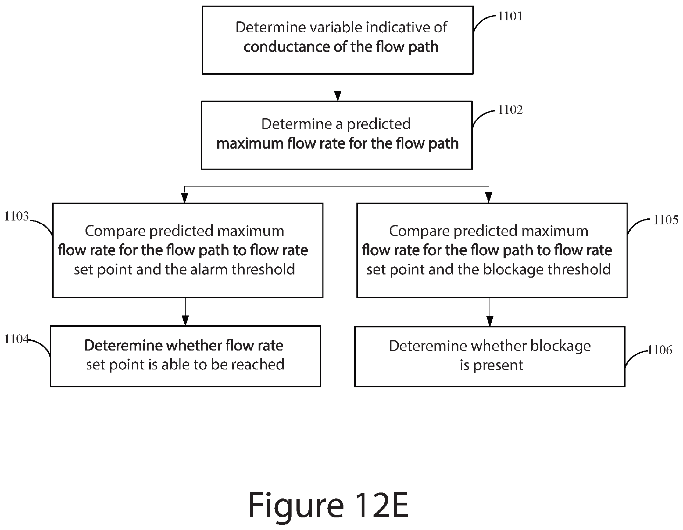

Figure 12E shows an example of a method of determining a cannot reach flow rate set point and a blockage alarm as performed by the controller 13 (both described in more detail above).

At step 1101, the controller 13 determines the variable indicative of conductance (as described in more detail above). Step 1101 may occur at each time step during the therapy by the controller 13 provided within the breathing apparatus.

At step 1102, the controller 13 determines the predicted maximum flow rate based on the variable indicative of conductance (calculated at step 1101) and the flow generator output range, and in this particular embodiment, the upper motor output of the allowable motor output range.

At step 1103, the predicted maximum flow is compared to the flow rate set point and an alarm threshold.

At step 1104, the controller 13 determines whether the flow rate set point is able to be reached, for example if the flow rate set point is greater than the predicted maximum flow rate by more than an alarm threshold, the controller 13 may generate an alarm condition (for example a cannot reach flow rate set point alarm).

At step 1105, the predicted maximum flow is compared to the flow rate set point and a blockage threshold.

At step 1106, the controller 13 determines whether a blockage is or may be present, in which case the controller 13 may generate a blockage alarm. For example, if the flow rate set point is greater than the predicted maximum flow rate by more than the blockage threshold, the controller 13 may generate a blockage alarm.

In some embodiments, the controller 13 may require multiple instances of the flow rate set point being greater than the predicted maximum flow rate by more than an alarm threshold (or a blockage threshold) in order to activate the relevant alarm. In these embodiments the controller 13 may require the number of consecutive "cannot reach set flow" or "blockage" events to exceed a consecutive-count threshold. If the consecutive-count threshold is exceeded, the controller 13 may then generate an alarm condition (for example a cannot reach flow rate set point alarm, or a blockage alarm).

Furthermore, in response to the generation of the at least one alarm condition as described above, the controller 13 may control the motor speed (or flow generator output) to a predetermined safe motor speed (or safe flow generator output).

In some embodiments, the controller 13 may control the motor speed (or flow generator output) to a predetermined safe motor speed (or safe flow generator output) when the blockage alarm is raised.

In some embodiments, the controller 13 may switch off the humidifier heating element 310, and/or the heating element 16a when an alarm condition is raised.

In some embodiments, the controller 13 may switch off the humidifier heating element 310, and/or the heating element 16a when the blockage alarm is raised.

In some embodiments, the controller 13 may control the valve to lower or prevent the flow of a supplemental gas (for example oxygen) via the valve when an alarm condition is raised.

The controller 13 may control the motor speed to a predetermined safe motor speed until the predicted maximum flow rate is greater than a safe threshold flow rate, and/or the predicted minimum flow rate is less than a safe threshold flow rate.

The controller 13 may control the motor speed to the predetermined safe motor speed until the flow rate set point is less than the predicted maximum flow rate, and/or the flow rate set point is greater than the predicted minimum flow rate.

The predetermined safe motor speed may be about 6000RPM to about 10000RPM or about 8000RPM.

The alarm condition may be triggered after one or a plurality of instances in which it is predicted that the apparatus cannot reach the flow rate set point.

Figure 13 shows an example of the above alarm conditions on a single graph. Figure 13 shows each of the cannot reach flow set point regions, and the blockage region. If the flow rate set point is in any of these regions, the corresponding alarm will be activated.

For example as shown in Figure 13 if the flow rate set point is greater than the alarm limit 903 (for example in the cannot reach flow set point region) a cannot reach flow set point and/or a flow rate set point too high alarm may be raised.

If the flow rate set point is greater than the blockage limit 904 (for example in the blockage region) a blockage alarm may be raised.

If the flow rate set point is less than the alarm limit 902 (for example in the cannot reach flow set point region) a cannot reach flow set point and/or a flow rate set point too low alarm may be raised.

It will be appreciated that the cannot reach flow rate set point alarm with respect to the predicted minimum flow rate may be given a different name (i.e. a flow rate set point too low) or generate a separate alarm condition (to the cannot reach flow set point alarm with respect to the predicted maximum flow rate).

It will be appreciated that the cannot reach flow rate set point alarm with respect to the predicted maximum flow rate may be given a different name (i.e. a flow rate set point too high).

The alarm condition may comprise one or more of: an audio alarm, and a visual alarm.

One or more thresholds (for example the alarm thresholds or blockage thresholds) may be varied based on a type of therapy being provided by the breathing apparatus (which could be, for example, a nasal high flow apparatus).

As described above the alarm threshold for use in comparison with the predicted maximum flow rate may be the same or different to the alarm threshold for use in comparison with the predicted minimum flow rate.

Also described is the comparison of a predicted flow rate for the flow path to a current flow rate to determine whether there has been a change in flow path conditions (e.g. a new leak or new blockage).

This approach, as described in more detail below, may be of particular use in systems with a large number of potential combinations of components. Each potential combination of components may result in a different flow path conductance and/or leak rate. For example, when using a high flow therapy device, each different non-sealing interface may have a different leak rate.

The system may be for use with different interfaces (for example different interface types or different interface sizes) and/or different conduits (for example for different therapies). There may be variability in how the components are used (for example differences in fit of the interface to a user) and variability in the system over the course of therapy (for example changes in conduit location, changes in condensate in the tube, and/or changes in the fit of the interface to a user).

Variability in patient interfaces may be an issue in systems with unsealed interfaces (for example high flow systems) due to the large range of interface sizes. For example, the largest interface (for example an adult large size interface) may have a resistance to flow orders of magnitude lower than the smallest interface (for example a neonatal interface). Therefore, prior art approaches may be ineffective because, as the range of resistances to flow across all interfaces is so large, one (or even several) preset thresholds will not allow for accurate detection of leak and/or blockages and/or connection or disconnection of the patient interface across the entire range of interfaces.

The system may be used with different patients that have different respiratory system structures and/or different respiratory system conditions (for example caused by illness.)

The system may therefore experience a wide variety of conditions and so prior art methods of determining leak and/or blockage may not work consistently across the entire range of conditions (without knowledge of at least some of the conditions of the system), and for example may alarm unnecessarily, or fail to alarm when it should.

Identifying components of the system may be time consuming for a clinician or introduce additional complexity to the components or system.

In the approach outlined below, to determine if there is a leak and/or blockage in the flow path, the system predicts the flow rate of the gases. The system then compares this predicted flow rate to the current, measured flow rate, or compares the current, measured flow rate to one or more thresholds (some of which may be based on the predicted flow rate). Because the predicted flow rate is a function of the conductance of the flow path (and, therefore, the combination of components), the accuracy with which the system can detect leaks and/or blockages is relatively unaffected by the combination of components being used. This is in contrast to prior art systems which may need to have information on, or make assumptions regarding, the particular components present.

The current flow rate may be the flow rate measured at the current time for the controller 13, or a recently-measured flow rate (e.g. a flow rate measured at a preceding time step). The current flow rate may be a filtered (or otherwise conditioned) output from a sensor. Alternatively, the current flow rate may be a raw output from a sensor (e.g. a flow sensor as described above).

The current flow rate being greater than the predicted flow rate (and/or a leak flow rate threshold) may indicate, for example, a leak, or removal of a component.

The current flow rate being less than the predicted flow rate (and/or a blockage flow rate threshold) may indicate, for example, a blockage, or occlusion of the tube (by condensate or kinking for example).

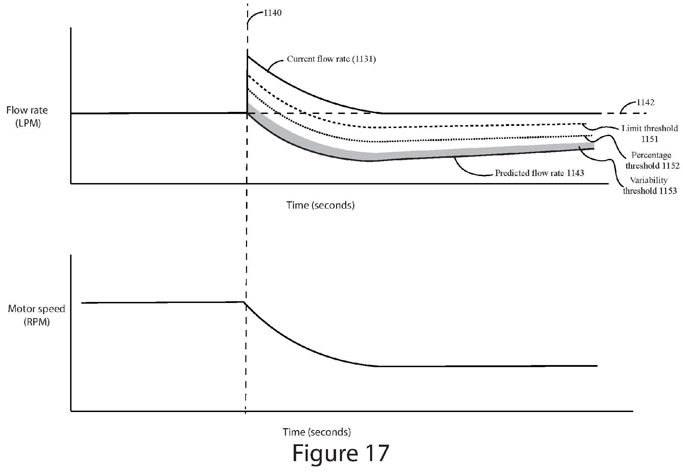

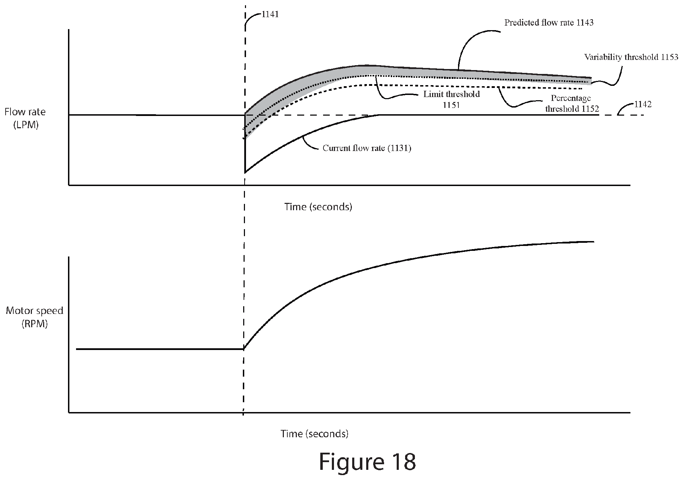

A flow rate threshold (as shown in Figures 17 and 18 for example), required to indicate a leak or blockage, or another flow path change event, may be variable over time. This variability may be due to the threshold being a function of a number of measurements in the system (for example, a historic value of a variable indicative of the conductance of the circuit).

The flow rate threshold may comprise one or more blockage flow rate thresholds (for determination of a blockage) and/or one or more leak flow rate thresholds (for determination of a leak).

The current flow rate may be compared to the blockage flow rate threshold when the predicted flow rate is greater than the current flow rate (for example as shown in Figure 18).

The current flow rate may be compared to the leak flow rate threshold when the predicted flow rate is less than the current flow rate (for example as shown in Figure 17).

In addition to undertaking comparisons for both leak and blockage flow rate thresholds it will be appreciated that the blockage flow rate threshold comparisons may be made without leak flow rate threshold comparisons, and vice versa.

In some embodiments, the controller 13 is configured to determine a predicted flow rate for the flow path.

The predicted flow rate for the flow path is based on the variable indicative of conductance of the flow path and a current flow generator output (as described in more detail above).

It will be appreciated the flow generator output may be the motor output (as described in more detail above). The below examples are related to the flow generator output as a motor output (as described in more detail above, for example a motor speed).

The controller 13 is configured to compare a current flow rate to the predicted flow rate, and, based on the comparison, generate at least one alarm condition.

Additionally, or alternatively, the controller 13 is configured to compare a current flow rate to a flow rate threshold that is a function of the predicted flow rate (for example at least one blockage flow rate threshold and/or at least one leak flow rate threshold), and may generate at least one alarm condition based on the comparison.

As described above, the at least one flow rate threshold may comprise at least one blockage flow rate threshold and/or at least one leak flow rate threshold.

The controller 13 is configured to generate at least one alarm condition (optionally a leak alarm condition) if the current flow rate is greater than the leak flow rate threshold.

The controller 13 is configured to generate at least one alarm condition if the current flow rate is greater than at least one leak flow rate threshold when the predicted flow rate is less than the current flow rate (i.e. indicative of a leak rather than a blockage).

The controller 13 is configured to generate at least one alarm condition (optionally a blockage alarm condition) if the current flow rate is less than at least one leak blockage rate threshold (for example as shown in Figure 18).

The controller 13 is configured to generate at least one alarm condition if the current flow rate is less than at least one blockage flow rate threshold when the predicted flow rate is greater than the current flow rate (i.e. indicative of a blockage rather than a leak).

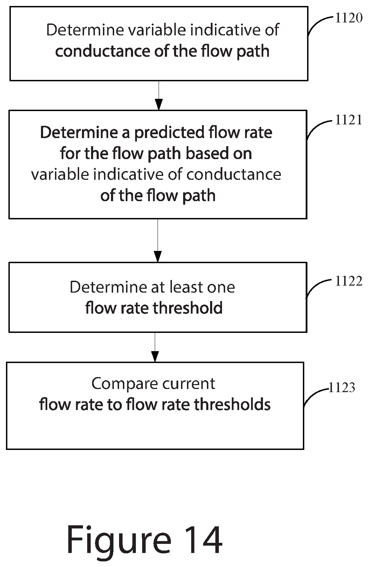

Figure 14 illustrates a method for determining a leak or blockage based on a predicted flow rate.

At step 1120, the controller 13 determines a variable indicative of the conductance of the flow path. As described in more detail below this may involve stored, historic information relating to the conductance of the flow path.

At step 1121, the controller 13 determines a predicted flow rate based on the variable indicative of the conductance of the flow path and the current flow generator output (for example a motor speed).

At step 1122, the controller 13 determines at least one flow rate threshold (as described in more detail below).

At step 1123, the controller compares the current flow rate to the flow rate thresholds. These flow rate thresholds may be blockage flow rate thresholds if the predicted flow rate is greater than the current flow rate or leak flow rate thresholds if the predicted flow rate is less than the current flow rate. The controller then generates at least one alarm condition if the current flow rate is greater than at least one (or optionally all) leak flow rate thresholds or less than at least one (or optionally all) blockage flow rate thresholds.

In response to the generation of the at least one alarm condition, the controller may control the motor speed to a predetermined safe motor speed. The controller 13 may control the motor speed to the predetermined safe motor speed until at least one alarm condition is cleared. The predetermined safe motor speed may be about 6000RPM to about 10000RPM or about 8000RPM (as described in more detail above).

In some embodiments, the controller 13 may switch off the humidifier heating element 310, and/or the heating element 16a when an alarm condition is raised.

In some embodiments, the controller 13 may control the valve to lower or prevent the flow of a supplemental gas (for example oxygen) via the valve when an alarm condition is raised.

In response to the generation of the at least one alarm condition, the controller may continue to attempt to provide therapy at a target flow rate.

The alarm condition comprises one or more of:
an audio alarm, and
a visual alarm.

The at least one alarm condition may be generated after a plurality of instances of the current flow rate being greater than at least one leak flow rate threshold.

The at least one alarm condition may be generated after the current flow rate is greater than at least one leak flow rate threshold for a threshold time period. The threshold time period may be about 0.5 seconds to about 5 seconds.

The at least one alarm condition may be generated after a plurality of instances of the current flow rate being less than at least one blockage flow rate threshold.

The at least one alarm condition may be generated after the current flow rate is less than at least one blockage flow rate threshold for a threshold time period. The threshold time period may be about 0.5 seconds to about 5 seconds.

The leak flow rate threshold and/or blockage flow rate threshold may comprise one or more of:
a percentage threshold based on a percentage of the predicted flow rate
a limit threshold based on the flow generator output and a constant,
a variability threshold based on a measurement of variability of the predicted flow rate.

Figures 15A and 15B show illustrations of the leak flow thresholds plotted with the predicted flow rate and the current flow rate.

In Figure 15A, the current flow rate is greater than all of the leak flow rate thresholds, and therefore an alarm condition is raised (for example a leak alarm).

In Figure 15B, the current flow rate is less than all of the leak flow rate thresholds, and therefore no alarm condition is raised.

Figures 15C and 15D shows illustrations of the blockage flow thresholds plotted with the predicted flow rate and the current flow rate.

In Figure 15C, the current flow rate is less than all of the blockage flow rate thresholds, and therefore an alarm condition (for example a blockage alarm) is raised.

In Figure 15D, the current flow rate is greater than all of the blockage flow rate thresholds, and therefore no alarm condition is raised.

It will be appreciated that the alarm condition may be generated based on the magnitude of the difference between the current flow rate and the predicted flow rate. However, in some configurations the controller 13 may take into account whether the difference between the current flow rate and the predicted flow rate is positive (i.e. the current flow rate is greater than the predicted flow rate, as shown in Figure 15A for example, which is indicative of a leak), or negative (i.e. the current flow rate is less than the predicted flow rate, as shown in Figure 15C for example, which is indicative of a blockage).

In some embodiments, an alarm condition is generated if the current flow rate is greater than all of the leak flow rate thresholds (i.e. the percentage threshold, the limit threshold and the variability threshold).

In some embodiments, an alarm condition is generated if the current flow rate is less than all of the blockage flow rate thresholds (i.e. the percentage threshold, the limit threshold and the variability threshold).

The percentage threshold, the limit threshold and the variability threshold may be the same, or different for the leak flow rate thresholds. The percentage threshold, the limit threshold and the variability threshold may be the same, or different for the blockage flow rate thresholds. It will be appreciated that the value of these thresholds may be different for the leak flow rate thresholds and the blockage flow rate thresholds.

The percentage threshold of the leak flow rate thresholds is about 105% to about 180%, or about 110% to about 120%, or about 150% to about 180% of the predicted flow rate.

The percentage threshold of the blockage flow rate thresholds is about 40% to about 95%, or about 50% to about 90%, or about 60% to about 80% of the predicted flow rate.

In some embodiments, the percentage threshold of the blockage flow rate thresholds and/or the leak flow rate thresholds may be based on the magnitude of the predicted flow.

The limit threshold may be based on the flow generator output and a constant. Alternatively, the limit threshold may be a constant.

In some embodiments the constant is a predetermined conductance (or, for example, another variable indicative of conductance). In other embodiments, the constant is a predetermined flow rate.

The limit threshold of the leak flow rate thresholds may use a first predetermined conductance and/or a first predetermined flow rate.

The limit threshold of the blockage flow rate thresholds may use a first predetermined conductance and/or a first predetermined flow rate or a second predetermined conductance and/or a second predetermined flow rate.

The variability threshold may be based on a standard deviation of the predicted flow rate.

The variability threshold of the leak flow rate thresholds may be the sum of the predicted flow rate and the standard deviation of the predicted flow rate.

The variability threshold of the blockage flow rate threshold may be the predicted flow rate minus the standard deviation of the predicted flow rate.

In some embodiments the variability threshold may be additionally or alternatively based on a standard deviation of the variable indicative of the conductance of the flow path.

The variability threshold may be a function of a variation in the predicted flow rate and/or a variation in the variable indicative of the conductance of the flow path and/or a variation in the flow generator output.

For example, if the variation in the predicted flow rate is large then the variability threshold may be large, and if the variation in the predicted flow rate is small then the variability threshold may be small.

The variability threshold may be based on stored motor speed data (for example where the variability threshold may be represented by a probability distribution function).

The variability threshold may be based on stored variable indicative of conductance data (for example where the variability threshold may be represented by a probability distribution function).

The variability threshold may be based on stored conductance data.

The variability threshold may be based on a noise associated with the variable indicative of conductance signal.

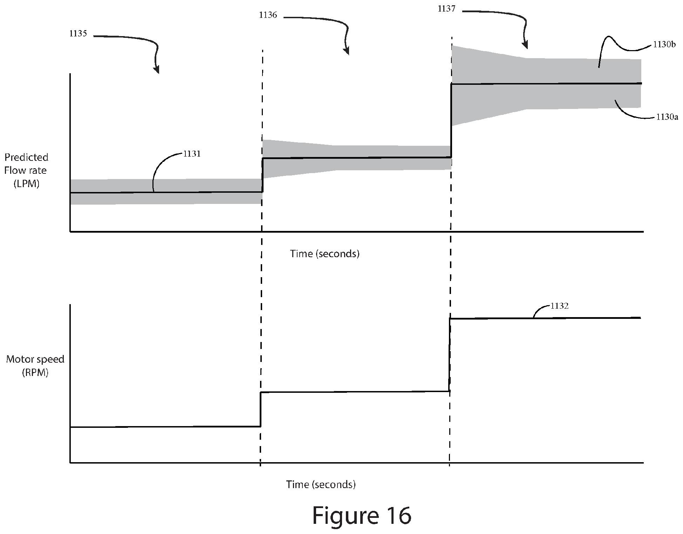

Figure 16 shows an example of a motor speed 1132 (as a flow generator output) and associated predicted flow rate 1131.

Figure 16 illustrates how the variability threshold may change in response to changes in the motor speed 1132. The variability threshold is plotted as a range around the predicted flow rate as both the variability threshold of the blockage flow rate threshold 1130a and the variability threshold of the leak flow rate threshold 1130b. The variability thresholds 1130a, 1130b increase in magnitude proportional to a corresponding change in motor speed 1132.With an increase in motor speed 1132, the predicted flow rate 1131 also increases in this example (as the conductance of the flow path remains constant.)

Each time the predicted flow rate increases (in response to an increase in motor speed 1132), the magnitude of the variability increases temporarily, before decreasing again.

The predicted flow rate is shown as changing in time with the motor speed 1132, however it will be appreciated that the predicted flow rate 1131 may lag the motor speed 1132.

In region 1135 the variability threshold or a variability uncertainty 1130 is reasonably stable.

In region 1136 there is a change in motor speed and so the standard deviation (or other probability measure) of the predicted flow rate increases, and so does the variability threshold 1130. As time progresses and the motor speed is stable, the variability threshold 1130 decreases and then stabilises.

In some embodiments the variability threshold 1130 is based on the magnitude of the predicted flow rate.

In some embodiments, the magnitude of the variability threshold 1130 is based on the standard deviation (or other probability measure) of the variable indicative of the conductance of the flow path.

The variability threshold may be proportional to a change in flow rate and/or motor speed and/or a variable indicative of conductance in a timing window. The timing window may be a predetermined time (for example the last 10 seconds).

As shown in Figure 16, the variability threshold may be greater nearer a recent change in motor speed. The variability threshold may then decrease to steady state as the motor speed settles to a steady state.

In region 1137 there is a larger change in motor speed relative to that in region 1136 and so the standard deviation (or other probability measure) of the variable indicative of the conductance of the flow path increases a greater amount than in region 1136, and so does the variability threshold. In regions 1136 and 1137, as time progresses and the motor speed is stable, the variability threshold 1130 decreases and then stabilises.

It will be appreciated that other variables indicative of statistical variation or error may be used in determining the variability threshold (for example variance, standard deviation, interquartile range, range, or mean absolute difference).

The variability threshold may be based on conductance data (as described in more detail below).

In some embodiments the variability threshold may be a function of (for example proportional to) the predicted flow rate. For example, the stabilised variability threshold 1130 in region 1137 is larger than that in regions 1135 and 1136 because the magnitude of the predicted flow rate is larger.

In some embodiments, the variability threshold may be a function of (for example proportional to) the amount of variation in the variable indicative of the conductance of the flow path.

Figure 17 shows an example of a cannula disconnection event 1140.

As shown in Figure 17, before the cannula disconnection event 1140 the current flow rate 1131 is provided at the set point flow rate 1142.

Once the cannula disconnection event 1140 occurs, the current flow rate 1131 increases due to the increased in conductance of the circuit, and the motor speed decreases to control the current flow rate to the flow rate set point. The predicted flow rate 1143 determined by the controller is shown as decreasing in line with the decrease in the motor speed.

Figure 17 illustrates a number of leak flow rate thresholds. For example, the limit threshold 1151, the percentage threshold 1152, and the variability threshold 1153.

Note the thresholds 1151, 1152, 1153 would be calculated before the event however the thresholds before the leak event are not shown in Figure 17.

The thresholds 1151, 1152, 1153 are shown as time continuous but are recalculated each time step and compared to the predicted flow. In this example, an alarm condition will be raised as the current flow rate is greater than the thresholds 1151, 1152, 1153, which is indicative of a leak.

Figure 18 shows an example of a blockage event 1140.

As shown in Figure 18, before the blockage event 1140 the current flow rate 1131 is provided at the set point flow rate 1142.

Once the blockage event 1141 occurs, the current flow rate 1131 decreases due to the decrease in conductance of the circuit, and the motor speed increase to control the current flow rate to the flow rate set point. The predicted flow rate 1143 determined by the controller is shown as increasing in line with the increase in the motor speed.

The controller 13 may select the blockage flow rate threshold.

Figure 18 illustrates a number of blockage flow rate thresholds. For example, the limit threshold 1151, the percentage threshold 1152, and the variability threshold 1153.

Note the thresholds 1151, 1152, 1153 would be calculated before the event however the thresholds before the blockage event are not shown in figure 18

The thresholds 1151, 1152, and 1153 are shown as time continuous but are recalculated each time step and compared to the predicted flow. In this example, an alarm condition will be raised as the current flow rate is less than the thresholds 1151, 1152, and 1153, which is indicative of a blockage.

In some embodiments, additionally or alternatively, a difference between a current flow rate and the predicted flow rate may be compared to a flow rate difference threshold. The flow rate difference threshold may be calculated based on the predicted flow rate. In these cases, instead of calculating a flow rate threshold and comparing this to the current flow rate, the difference between the current flow rate and a predicted flow rate is calculated and compared to a flow rate difference threshold.

The flow rate difference threshold may be determined based on the current flow rate and the flow rate threshold (as described below).

The controller 13 may undertake the process described above in a time step process. For example, as described below.

In some embodiments the controller 13 may, at a first time step, determine a variable indicative of conductance of the flow path (or, for example, update the variable indicative of conductance of the flow path based on a new measurement).

In some embodiments the controller 13 may at a second time step, be configured to determine a predicted flow rate based on the variable indicative of conductance of the flow path determined in the first time step.

In some embodiments, at each time step, the variable indicative of conductance of the flow path may be updated based on the variable indicative of conductance at the current time step, and the variable indicative of conductance at the previous time step.

In some embodiments, at each time step, the variable indicative of conductance of the flow path may be updated based on the current flow generator output and the current flow rate.

In some embodiments, at each time step, the variable indicative of conductance of the flow path may be updated based on a number of weighting factors. For example, in one embodiment, one of the weighting factors may be based on the measurement noise associated with one or more of, the variable indicative of conductance at the current time step, the flow rate, and the flow generator output.

The variable indicative of conductance of the flow path may be determined in a way as described elsewhere in the specification.

The variable indicative of conductance of the flow path may be represented by a probability density function. For example, the variable indicative of conductance of the flow path may be (or may comprise) a function indicating the relative likelihood that the variable indicative of conductance takes a certain value. The features of this probability density function may be used in determining the variability threshold (or any other threshold).

In some embodiments, the function to update the variable indicative of conductance of the flow path (as represented by a probability density function) may be based on the weighting factors as described above.

The variable indicative of conductance of the flow path may be based on stored data. The stored data may comprise at least one historic determination of one or more measured or determined variables (for example, the variable indicative of conductance (which may for example be conductance), flow rate, pressure, and motor output.)

In some embodiments, the stored data may be conductance data and comprise historic determinations of the variable indicative of conductance (for example conductance of the flow path). In these embodiments it will be appreciated that the variable indicative of conductance as part of the conductance data may be different or the same type of variable indicative of conductance used by the system in determining a predicted flow (as described above).

The controller 13 may store at least one historic determination of one or more measured or determined variables, in memory and use the at least one historic determination to calculate the variable indicative of conductance at the present time. In this way the variable indicative of conductance of the flow path may be dynamically updated based on previous values of one or more one or more measured or determined variables.

The variable indicative of conductance of the flow path may be based on stored data and weighting factors as described above. The variable indicative of conductance of the flow path may be based on stored data of the flow path over a timing window. For example, the controller 13 may calculate the variable indicative of conductance of the flow path based on the stored data from within a preceding timing window. The timing window may be, for example, a preceding 20 seconds.

At each time step, or periodically, the determination of a conductance of the flow path may be stored as the at least one historic determination of the conductance of the flow path.

The stored data may comprise timestamp information associated with a respective historic determination of the one or more measured or determined variables. This time stamp information may be used by the controller 13 in determining the variable indicative of conductance of the flow path.

The timestamp information may comprise a time that has elapsed since the historic determination of the one or more measured or determined variables.

The controller 13, may weight the stored data in the determination of the conductance of the flow path based on the associated timestamp information.

In some embodiments, more recent stored data is given a larger weighting than less recent conductance data.

The conductance data may comprise a weighting factor associated with each respective historic determination of one or more measured or determined variables.

The controller may be configured to determine a conductance of the flow path, and to determine a predicted flow rate for the flow path continuously, or periodically.

In some embodiments, the controller is configured to determine a predicted flow rate uncertainty.

As described above, the variable indicative of a conductance of the flow path may for example be defined by a value, or a number of values (i.e. defining for example a probability distribution, or a vector) for the variable indicative of conductance. When determining the variable indicative of a conductance of the flow path the controller may for example determine the value or number of values.

It will be appreciated that any list in the specification also discloses all combinations of items in the list.

Various examples and aspects may be provided by the following numbered clauses:
Numbered clause 1. A breathing apparatus, the breathing apparatus comprising:
   a flow generator,
   a flow path, the flow path configured to provide a flow of breathable gas to a patient,
   a controller, the controller configured to control the flow generator by controlling a flow generator output to the flow generator, wherein the flow generator output is within an allowable flow generator output range, to provide the flow of breathable gas, and
   wherein the controller is configured to:
      determine a variable indicative of conductance of the flow path,
      determine a predicted maximum flow rate and/or a predicted minimum flow rate for the flow path based on:
         the variable indicative of conductance of the flow path,
         the allowable flow generator output range.
Numbered clause 2. The breathing apparatus of numbered clause 1, wherein the flow generator output is or comprises:
   i. a motor speed,
   ii. a motor current,
   iii. a motor duty,
   iv. a pressure of the gases in the flow path, or
   v. any combination of i-iv.
Numbered clause 3. The breathing apparatus of numbered clause 1 or numbered clause 2, wherein the variable indicative of conductance of the flow path is, or is based on:
   i. a conductance of the flow path, or a portion of the flow path;
   ii. a resistance to flow of the flow path, or a portion of the flow path; or
   iii. a pressure drop along the flow path, or a portion of the flow path
   iv. any combination of i-iii.
Numbered clause 4. The breathing apparatus of any one of numbered clauses 1 to 3, wherein the flow generator comprises an impeller.
Numbered clause 5. The breathing apparatus of any one of numbered clauses 1 to 4, wherein the breathing apparatus comprises a flow sensor, wherein the flow sensor is configured to measure a flow rate of the flow of breathable gas to a patient.
Numbered clause 6. The breathing apparatus of any one of numbered clauses 1 to 5, wherein the variable indicative of conductance of the flow path is based on a variable indicative of conductance of the flow path at a previous time step or, wherein the variable indicative of conductance of the flow path is based on a variable indicative of conductance of the flow path over a timing window, optionally the timing window is a preceding 20 seconds.
Numbered clause 7. The breathing apparatus of any one of numbered clauses 1 to 6, wherein the variable indicative of conductance of the flow path is based on the current flow generator output and a current flow rate.
Numbered clause 8. The breathing apparatus of any one of numbered clauses 1 to 7, wherein the variable indicative of conductance of the flow path is based on the current flow divided by the current flow generator output.
Numbered clause 9. The breathing apparatus of any one of numbered clauses 1 to 8, wherein the variable indicative of conductance is proportional to: $\frac{flow rate}{\sqrt{pressure}}$
Numbered clause 10. The breathing apparatus of any one of numbered clauses 1 to 9, wherein the variable indicative of conductance is proportional to: $\frac{flow rate}{motor output}$
Numbered clause 11. The breathing apparatus of any one of numbered clauses 1 to 10, wherein the flow path comprises one or more of:
   a humidification chamber,
   one or more breathing conduits,
   a patient interface,
   an elbow, and
   a sensor module.
Numbered clause 12. The breathing apparatus of any one of numbered clauses 1 to 11, wherein the controller configured to control the motor to provide, or attempt to provide, the flow of breathable gas to the patient at a flow rate set point.
Numbered clause 13. The breathing apparatus of any one of numbered clauses 1 to 12, wherein the allowable flow generator output range is an allowable motor speed range, wherein the allowable motor speed range comprises an upper motor speed limit and/or a lower motor speed limit.
Numbered clause 14. The breathing apparatus of numbered clause 13, wherein the predicted maximum flow rate is based on the current motor speed and/or an upper motor speed limit.
Numbered clause 15. The breathing apparatus of numbered clause 13 or numbered clause 14, wherein the predicted minimum flow rate is based on the current motor speed and/or a lower motor speed limit.
Numbered clause 16. The breathing apparatus of any one of numbered clauses 1 to 15, wherein the controller is configured to compare the predicted maximum flow rate and/or the predicted minimum flow rate, and a flow rate set point, and based on the comparison generate at least one alarm condition.
Numbered clause 17. The breathing apparatus of numbered clause 16, wherein the at least one alarm condition comprises one or more of:
   a cannot reach flow rate set point alarm, and
   a blockage alarm.
Numbered clause 18. The breathing apparatus of numbered clause 17, wherein the cannot reach flow rate set point alarm comprises:
   a cannot reach flow rate alarm associated with the flow rate set point being above the maximum predicted flow rate (i.e. a flow rate set point too high alarm), and/or
   a cannot reach flow rate alarm associated with the flow rate set point being below the minimum predicted flow rate (i.e. a flow rate set point too low alarm).
Numbered clause 19. The breathing apparatus of numbered clause 17 or 18, wherein the controller is configured to determine a condition where the flow rate set point cannot be reached, based on the predicted maximum flow rate and the flow rate set point, and in response, the controller is configured to generate a cannot reach flow rate set point alarm.
Numbered clause 20. The breathing apparatus of any one of numbered clauses 16 to 19, wherein the controller is configured to generate at least one alarm condition when the flow rate set point is greater than the predicted maximum flow rate.
Numbered clause 21. The breathing apparatus of any one of numbered clauses 16 to 20, wherein the controller is configured to generate at least one alarm condition when the flow rate set point is greater than the predicted maximum flow rate by more than an alarm threshold.
Numbered clause 22. The breathing apparatus of numbered clause 21, wherein the alarm threshold is a proportion of the flow rate set point.
Numbered clause 23. The breathing apparatus of numbered clause 22, wherein the proportion of the flow rate set point is about 5% to about 20% or about 10% of the flow rate set point.
Numbered clause 24. The breathing apparatus of any one of numbered clauses 17 to 23, wherein the controller is configured to determine a condition where flow rate set point cannot be reached, based on the predicted minimum flow rate and the flow rate set point, and in response the controller is configured to generate a cannot reach flow rate set point alarm.
Numbered clause 25. The breathing apparatus of any one of numbered clauses 17 to 24, wherein the controller is configured to generate at least one alarm condition when the flow rate set point is less than the predicted minimum flow rate .
Numbered clause 26. The breathing apparatus of any one of numbered clauses 17 to 25, wherein the controller is configured to generate at least one alarm condition when the flow rate set point is less than the predicted minimum flow rate by more than an alarm threshold.
Numbered clause 27. The breathing apparatus of numbered clause 26, wherein the alarm threshold is a proportion of the flow rate set point.
Numbered clause 28. The breathing apparatus of numbered clause 27, wherein the proportion of the flow rate set point is about 5% to about 20% or about 10% of the flow rate set point.
Numbered clause 29. The breathing apparatus of any one of numbered clauses 16 to 28, wherein in response to the generation of the at least one alarm condition, the controller controls the motor speed to a predetermined safe motor speed.
Numbered clause 30. The breathing apparatus of numbered clause 29, wherein the controller controls the motor speed to the predetermined safe motor speed until the predicted maximum flow rate is greater than a safe threshold flow rate, and/or the predicted minimum flow rate is less than a safe threshold flow rate.
Numbered clause 31. The breathing apparatus of numbered clause 29 or numbered clause 30, wherein the controller controls the motor speed to the predetermined safe motor speed until the flow rate set point is less than the predicted maximum flow rate, and/or the flow rate set point is greater than the predicted minimum flow rate.
Numbered clause 32. A breathing apparatus, the breathing apparatus comprising:
   a flow generator,
   a flow path, the flow path configured to provide a flow of breathable gas to a patient,
   a controller, the controller configured to control the flow generator by controlling a flow generator output to the flow generator, to provide the flow of breathable gas to a patient, and
      wherein the controller is configured to:
      determine a variable indicative of conductance of the flow path, and
      determine a predicted flow rate for the flow path based on:
         the variable indicative of conductance of the flow path and
         the current flow generator output.
Numbered clause 33. The breathing apparatus of numbered clause 32, wherein at a first time step, the controller is configured to determine a variable indicative of conductance of the flow path.
Numbered clause 34. The breathing apparatus of numbered clause 33, wherein at a second time step, the controller is configured to determine a predicted flow rate based on the variable indicative of conductance of the flow path.
Numbered clause 35. The breathing apparatus of any one of numbered clauses 32 to 34, wherein the flow generator output is or comprises:
   i. a motor speed,
   ii. a motor current,
   iii. a motor duty,
   iv. a pressure of the gases in the flow path, or
   v. any combination of i-iv.
Numbered clause 36. The breathing apparatus of any one of numbered clauses 32 to 35, wherein the controller is configured to compare a current flow rate and the predicted flow rate, and based on the comparison generate at least one alarm condition.
Numbered clause 37. The breathing apparatus of any one of numbered clauses 32 to 36, wherein the controller is configured to generate a leak alarm condition if the current flow rate is greater than at least one leak flow rate threshold (optionally when the predicted flow rate is less than the current flow rate).
Numbered clause 38. The breathing apparatus of numbered clause 37, wherein the leak alarm condition is generated when the current flow rate is greater than all of the at least one leak flow rate threshold.
Numbered clause 39. The breathing apparatus of numbered clause 37 or numbered clause 38, wherein the at least one leak flow rate threshold comprises one or more of:
   a percentage threshold based on a percentage of the predicted flow rate
   a limit threshold based on the flow generator output and optionally a constant,
   a variability threshold based on the variability of the predicted flow rate.
Numbered clause 40. The breathing apparatus of any one of numbered clauses 32 to 39, wherein the controller is configured to generate at least one blockage alarm condition if the current flow rate is less than the at least one blockage flow rate threshold.
Numbered clause 41. The breathing apparatus of numbered clause 40, wherein the blockage alarm condition is generated when the current flow rate is less than all of the at least one blockage flow rate threshold.
Numbered clause 42. The breathing apparatus of numbered clause 40 or numbered clause 41, wherein the at least one blockage flow rate threshold comprises one or more of:
   a percentage threshold based on a percentage of the predicted flow rate
   a limit threshold based on the flow generator output and optionally a constant,
   a variability threshold based on the variability of the predicted flow rate.
Numbered clause 43. The breathing apparatus of numbered clause 42, wherein if the current flow rate is less than any combination of the percentage threshold, the limit threshold and/or the variability threshold, the controller is configured to generate the blockage alarm condition.
Numbered clause 44. The breathing apparatus of any one of numbered clauses 32 to 43, wherein in response to the generation of the at least one blockage alarm condition and/or the at least one leak alarm condition, the controller controls the motor speed to a predetermined safe motor speed.
Numbered clause 45. The breathing apparatus of numbered clause 44, wherein the controller controls the motor speed to the predetermined safe motor speed until at least one leak alarm condition or the at least one blockage alarm condition is cleared.
Numbered clause 46. The breathing apparatus of any one of numbered clauses 37 to 45, wherein the leak alarm condition and/or the blockage alarm condition comprises one or more of:
   an audio alarm, and
   a visual alarm.
Numbered clause 47. The breathing apparatus of any one of numbered clauses 32 to 46, wherein the controller is configured to determine a conductance of the flow path and determine a predicted flow rate for the flow path continuously, or periodically.

## Claims

1. A breathing apparatus, the breathing apparatus comprising:
a flow generator,
a flow path, the flow path configured to provide a flow of breathable gas to a patient,
a controller, the controller configured to control the flow generator by controlling a flow generator output to the flow generator, to provide the flow of breathable gas to a patient, and
wherein the controller is configured to:
determine a variable indicative of conductance of the flow path, and
determine a predicted flow rate for the flow path based on:
the variable indicative of conductance of the flow path and
the current flow generator output.

2. The breathing apparatus of claim 1, wherein at a first time step, the controller is configured to determine a variable indicative of conductance of the flow path, and at a second time step, the controller is configured to determine a predicted flow rate based on the variable indicative of conductance of the flow path.

3. The breathing apparatus of claim 1 or claim 2, wherein the flow generator output is or comprises:
i. a motor speed,
ii. a motor current,
iii. a motor duty,
iv. a pressure of the gases in the flow path, or
v. any combination of i-iv.

4. The breathing apparatus of any one of claims 1 to 3, wherein the controller is configured to compare a current flow rate and the predicted flow rate, and based on the comparison generate at least one alarm condition.

5. The breathing apparatus of any one of claims 1 to 4, wherein the controller is configured to generate a leak alarm condition if the current flow rate is greater than at least one leak flow rate threshold (optionally when the predicted flow rate is less than the current flow rate).

6. The breathing apparatus of claim 5, wherein the leak alarm condition is generated when the current flow rate is greater than all of the at least one leak flow rate threshold.

7. The breathing apparatus of claim 5 or claim 6, wherein the at least one leak flow rate threshold comprises one or more of:
a percentage threshold based on a percentage of the predicted flow rate
a limit threshold based on the flow generator output and optionally a constant,
a variability threshold based on the variability of the predicted flow rate.

8. The breathing apparatus of any one of claims 1 to 7, wherein the controller is configured to generate at least one blockage alarm condition if the current flow rate is less than the at least one blockage flow rate threshold.

9. The breathing apparatus of claim 8, wherein the blockage alarm condition is generated when the current flow rate is less than all of the at least one blockage flow rate threshold.

10. The breathing apparatus of claim 8 or claim 9, wherein the at least one blockage flow rate threshold comprises one or more of:
a percentage threshold based on a percentage of the predicted flow rate
a limit threshold based on the flow generator output and optionally a constant,
a variability threshold based on the variability of the predicted flow rate.

11. The breathing apparatus of claim 10, wherein if the current flow rate is less than any combination of the percentage threshold, the limit threshold and/or the variability threshold, the controller is configured to generate the blockage alarm condition.

12. The breathing apparatus of any one of claims 1 to 11, wherein in response to the generation of the at least one blockage alarm condition and/or the at least one leak alarm condition, the controller controls the motor speed to a predetermined safe motor speed.

13. The breathing apparatus of claim 12, wherein the controller controls the motor speed to the predetermined safe motor speed until at least one leak alarm condition or the at least one blockage alarm condition is cleared.

14. The breathing apparatus of any one of claims 5 to 13, wherein the leak alarm condition and/or the blockage alarm condition comprises one or more of:
an audio alarm, and
a visual alarm.

15. The breathing apparatus of any one of claims 1 to 14, wherein the controller is configured to determine a conductance of the flow path and determine a predicted flow rate for the flow path continuously, or periodically.
